# EUROPEAN PATENT APPLICATION

(11) **EP 2 113 258 A1**
(43) Date of publication of application: **04.11.2009**
(21) Application number: 07830664.4
(22) Date of filing: 26.10.2007
(51) Int. Cl.: A61K 51/00, C07D 307/83

(54) **AURONE DERIVATIVE-CONTAINING COMPOSITION FOR DIAGNOSIS**

(30) Priority: 05.12.2006 JP 2006328131; 27.03.2007 JP 2007081602
(71) Applicant: Nagasaki University, Nagasaki-Shi, Nagasaki 852-8521 (JP)
(72) Inventor: ONO, Masahiro, Kyoto 6110002 (JP)
(74) Representative: Spaltmann, Frank
(86) International application number: PCT/JP2007/070930
(87) International publication number: WO 2008/068974

(57) **Abstract**

The present invention provides a composition for diagnosing amyloid-related diseases, comprising a compound represented by general formula (I): 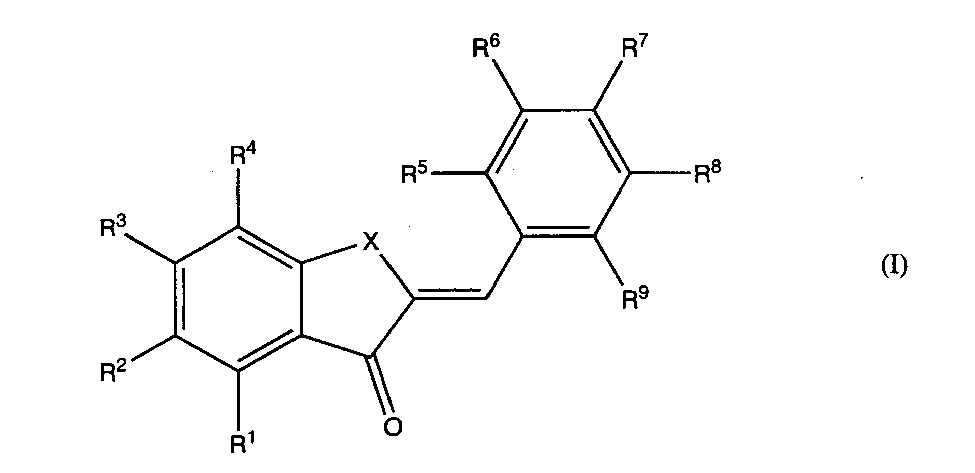 wherein X represents O or the like; R¹, R³, R⁴, R⁵, R⁶, R⁸ and R⁹ represent a hydrogen atom or the like; R² represents a halogen atom or the like; and R⁷ represents a dimethylamino group or the like;
or the compound labeled with a radionuclide, or a pharmaceutically acceptable salt of the compound or the labeled compound. The compound represented by general formula (I) has all of the following properties: high binding specificity to amyloid β protein, high permeability through the blood-brain barrier, and rapid clearance from sites without senile plaques in the brain.

## Description

### TECHNICAL FIELD

The present invention relates to an aurone derivative-containing composition for use in diagnosis. The composition of the present invention may be used for diagnosing amyloid-related diseases such as Alzheimer's disease.

### BACKGROUND ART

With the rapid aging of population in recent years, increases in the number of patients with diseases associated with dementia such as Alzheimer's disease (AD) have become one of serious social problems. Currently, the Hasegawa Scale, ADAS, and MMSE are available as methods for clinical diagnosis of AD. All of these methods quantitatively evaluate the decreased cognitive function in an individual suspected of AD and are commonly used. In addition, image diagnosis methods (MRI, CT, etc.) are supplementarily used. However, these diagnostic methods are inadequate for definite diagnosis of AD, and development of senile plaques and neurofibrils needs to be confirmed in biopsy of the brain before death or histopathological examination of the brain after death to make definite diagnosis. Accordingly, it is difficult to diagnose AD at an early stage before occurrence of an extensive brain damage by the current diagnostic methods. Although some reports have shown biological diagnostic markers for AD so far, no clinically practical technique has yet been developed. Under such circumstances, social demand for early diagnosis of AD is high, and urgent development of a method therefor is eagerly anticipated.

Senile plaques are the most characteristic brain lesion of AD, and the major component thereof is amyloid β protein having a β-sheet structure. Imaging of senile plaques from out of the body is considered to lead to the establishment of an effective diagnostic method for AD, and such imaging requires a probe compound that specifically binds to amyloid β protein. So far, several derivatives containing congo red or thioflavine T as a parent structure have been reported as probe compounds (Japanese Patent Unexamined Publications No. 2004-250407 and No. 2004-250411; Klunk W.E. et al., Annals of Neurology Vol. 55, No. 3, March 2004, 306-319), but these compounds suffer from many problems including low specificity to amyloid β protein, low permeability through the blood-brain barrier, and slow clearance due to their nonspecific binding in the brain. Therefore, these reported compounds have not yet been put into practical use for diagnosis of diseases associated with accumulation of amyloid at present. The present inventors have found that flavone derivatives, chalcone derivatives, styrylchromone derivatives and coumarin derivatives specifically bind to amyloid β protein and already filed a patent application based on this finding (Patent Document 1).
[Patent Document 1] WO/2006/057323

### DISCLOSURE OF THE INVENTION

### Problem for Solution by the Invention

The present invention was accomplished under the technical background described above, and an object thereof is to provide a compound having the following three properties: high binding specificity to amyloid β protein, high permeability through the blood-brain barrier, and rapid clearance from sites without senile plaques in the brain.

### Means to Solve the Problem

As a result of intensive and extensive researches toward the solution of the above problem, the present inventors have found that aurone derivatives have an excellent property as a probe compound for imaging amyloid β protein, and achieved the present invention based on this finding.

Specifically, the present invention provides the following (1) to (11).
(1) A composition for diagnosing amyloid-related diseases, comprising a compound represented by general formula (I): 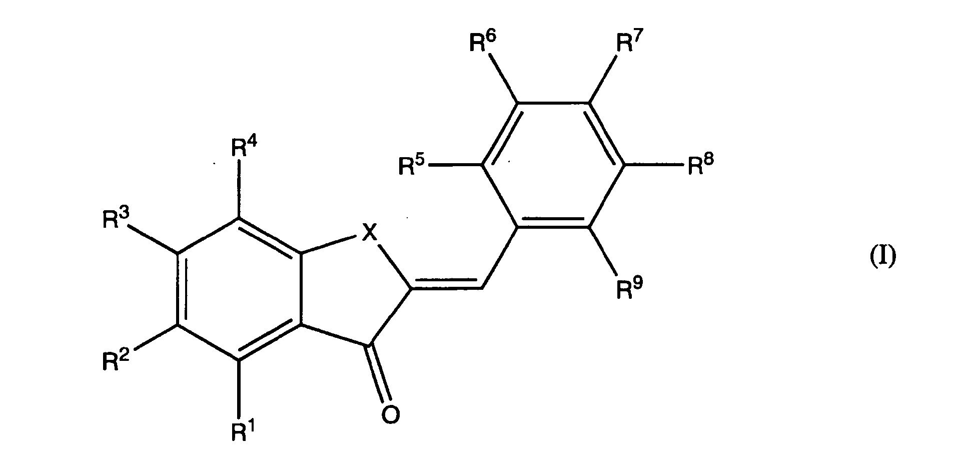 wherein X represents O or NH; R¹, R², R³ and R⁴ are the same or different and each represent a hydrogen atom, a halogen atom, a group represented by a formula -(CH₂CH₂O)ₙ-F where n is an integer from 1 to 10 or a group represented by a formula -(CH₂CH₂O)ₙ-OH where n is an integer from 1 to 10; and R⁵, R⁶, R⁷, R⁸ and R⁹ are the same or different and each represent a hydrogen atom, a halogen atom, a dimethylamino group, a methylamino group, an amino group, a methoxy group, a hydroxyl group, a group represented by a formula -(CH₂CH₂O)ₙ-F where n is an integer from 1 to 10 or a group represented by a formula -(CH₂CH₂O)ₙ-OH where n is an integer from 1 to 10;
   or the compound labeled with a radionuclide, or a pharmaceutically acceptable salt of the compound or the labeled compound.
(2) The composition according to (1) above, wherein X in general formula (I) is O.
(3) The composition according to (1) or (2) above, wherein R² in general formula (I) is a fluorine atom, an iodine atom or a group represented by a formula -(CH₂CH₂O)ₙ-F where n is an integer from 1 to 10.
(4) The composition according to (1) or (2) above, wherein R² in general formula (I) is a fluorine atom, an iodine atom or a group represented by a formula -(CH₂CH₂O)ₙ-F where n is an integer from 1 to 10; and R¹, R³ and R⁴ each represent a hydrogen atom.
(5) The composition according to any one of (1) to (4) above, wherein R⁷ in general formula (I) is a fluorine atom, an iodine atom, a dimethylamino group, a methylamino group, an amino group, a methoxy group, a hydroxyl group, a group represented by a formula -(CH₂CH₂O)ₙ-F where n is an integer from 1 to 10 or a group represented by a formula -(CH₂CH₂O)ₙ-OH where n is an integer from 1 to 10.
(6) The composition according to any one of (1) to (4) above, wherein R⁷ in general formula (I) is a fluorine atom, an iodine atom, a dimethylamino group, a methylamino group, an amino group, a methoxy group, a hydroxyl group, a group represented by a formula -(CH₂CH₂O)ₙ-F where n is an integer from 1 to 10 or a group represented by a formula -(CH₂CH₂O)ₙ-OH where n is an integer from 1 to 10; and R⁵, R⁶, R⁸ and R⁹ each represent a hydrogen atom.
(7) The composition according to any one of (1) to (6) above, wherein the radionuclide is a positron-emitting radionuclide.
(8) The composition according to any one of (1) to (6) above, wherein the radionuclide is a γ-ray-emitting radionuclide.
(9) The composition according to any one of (1) to (8) above, wherein the amyloid-related disease is Alzheimer's disease.
(10) A method of screening for therapeutic or prophylactic agents for amyloid-related diseases, comprising a step of administering a test substance to an amyloid-related disease model animal, a step of administering the composition according to any one of (1) to (9) above to the model animal, and a step of examining the distribution or quantity of the compound represented by general formula (I) contained in the brain of the model animal.
(11) A method of evaluating therapeutic or prophylactic agents for amyloid-related diseases, comprising a step of administering a therapeutic or prophylactic agent for amyloid-related diseases to an amyloid-related disease model animal, a step of administering the composition according to any one of (1) to (9) above to the model animal, and a step of examining the distribution or quantity of the compound represented by general formula (I) contained in the brain of the model animal.

### EFFECT OF THE INVENTION

Since the compound represented by general formula (I) has high binding specificity to amyloid β protein and a property of being rapidly eliminated from sites other than senile plaques in the brain, the compound is useful in diagnosing Alzheimer's disease.

### BRIEF DESCRIPTION OF THE DRAWINGS

Fig. 1 shows a process of synthesizing aurone derivatives with dimethylamino group or the like (1). Numbers in this figure correspond to compound numbers.
Fig. 2 shows a process of synthesizing aurone derivatives with dimethylamino group or the like (2). Numbers in this figure correspond to compound numbers.
Fig. 3 shows a process of synthesizing aurone derivatives with dimethylamino group or the like (3). Numbers in this figure correspond to compound numbers.
Fig. 4 shows the binding properties of [¹²⁵I]-labeled aurone derivatives to Ab(1-42) aggregate (1).
Fig. 5 shows the binding properties of [¹²⁵I]-labeled aurone derivatives to Ab(1-42) aggregate (2).
Fig. 6 shows the results of autoradiography and immunostaining on hippocampal sections from an Alzheimer's disease patient.
Fig. 7 shows a process of synthesizing aurone derivatives having hydroxyethoxy group or the like (1). Numbers in this figure correspond to compound numbers.
Fig. 8 shows a process of synthesizing aurone derivatives having hydroxyethoxy group or the like (2). Numbers in this figure correspond to compound numbers.
Fig. 9 shows the results of immunostaining on brain sections from an Alzheimer's disease model mouse (1: compound 26; 2: thioflavin S; 3: anti-amyloid antibody).
Fig. 10 shows the results of autoradiography and immunostaining on hippocampal sections from an Alzheimer's disease patient (A: autoradiography with [¹²⁵I]26; B: positive site in immunostaining with anti-amyloid β(1-42) antibody; C: negative site in immunostaining with anti-amyloid β(1-42) antibody).

### BEST MODE FOR CARRYING OUT THE INVENTION

Hereinbelow, the present invention will be described in details.

The term "halogen atom" used herein refers to, for example, fluorine atom, chlorine atom, bromine atom or iodine atom.

In a formula -(CH₂CH₂O)ₙ-F, n is preferably an integer from 1 to 3.

In a formula -(CH₂CH₂O)ₙ-OH, n is preferably an integer from 1 to 3.

In general formula (I), X is preferably O.

In general formula (I), R¹ is preferably a hydrogen atom.

In general formula (I), R² is preferably a fluorine atom, an iodine atom or a group represented by a formula -(CH₂CH₂O)ₙ-F where n is an integer from 1 to 10; more preferably, R² is a fluorine atom, an iodine atom, a 2-fluoroethoxy group, a 2-(2-fluoroethoxy)ethoxy group or a 2-{2-(2-fluoroethoxy)ethoxy}ethoxy group.

In general formula (I), R³ is preferably a hydrogen atom.

In general formula (I), R⁴ is preferably a hydrogen atom.

In general formula (I), R⁵ is preferably a hydrogen atom.

In general formula (I), R⁶ is preferably a hydrogen atom.

In general formula (I), R⁷ is preferably a fluorine atom, an iodine atom, a dimethylamino group, a methylamino group, an amino group, a methoxy group, a hydroxyl group, a group represented by a formula -(CH₂CH₂O)ₙ-F where n is an integer from 1 to 10 or a group represented by a formula -(CH₂CH₂O)ₙ-OH where n is an integer from 1 to 10; more preferably, R⁷ is a fluorine atom, an iodine atom, a dimethylamino group, a methylamino group, an amino group, a methoxy group, a hydroxyl group, a 2-fluoroethoxy group, a 2-(2-fluoroethoxy)ethoxy group, a 2-{2-(2-fluoroethoxy)ethoxy}ethoxy group, a 2-hydroxyethoxy group, a 2-(2-hydroxyethoxy)ethoxy group or a 2-{2-(2-hydroxyethoxy)ethoxy}ethoxy group; still more preferably, R⁷ is a dimethylamino group, a methylamino group, a 2-hydroxyethoxy group, a 2-(2-hydroxyethoxy)ethoxy group or a 2-{2-(2-hydroxyethoxy)ethoxy}ethoxy group.

In general formula (I), R⁸ is preferably a hydrogen atom.

In general formula (I), R⁹ is preferably a hydrogen atom.

Representative examples of those compounds represented by general formula (I) are shown in the following Tables. In the Tables, "Me" represents methyl group; "FX" represents 2-fluoroethoxy group; "FX2" represents 2-(2-fluoroethoxy)ethoxy group; "FX3" represents 2-{2-(2-fluoroethoxy)ethoxy}ethoxy group; "HOX" represents 2-hydroxyethoxy group; "HOX2" represents 2-(2-hydroxyethoxy)ethoxy group; and "HOX3" represents 2-{2-(2-hydroxyethoxy)ethoxy}ethoxy group.

**Table 1**

| | X | R¹ | R² | R³ | R⁴ | R⁵ | R⁶ | R⁷ | R⁸ | R⁹ |
|---|---|---|---|---|---|---|---|---|---|---|
| I-1 | O | H | H | F | H | H | H | H | H | H |
| I-2 | O | H | H | F | H | H | H | NH₂ | H | H |
| I-3 | O | H | H | F | H | H | H | OMe | H | H |
| I-4 | O | H | H | F | H | H | H | OH | H | H |
| I-5 | O | H | H | I | H | H | H | H | H | H |
| I-6 | O | H | H | I | H | H | H | NH₂ | H | H |
| I-7 | O | H | H | I | H | H | H | OMe | H | H |
| I-8 | O | H | H | I | H | H | H | OH | H | H |
| I-9 | O | H | H | FX | H | H | H | H | H | H |
| I-10 | O | H | H | FX | H | H | H | NH₂ | H | H |
| I-11 | O | H | H | FX | H | H | H | OMe | H | H |
| I-12 | O | H | H | FX | H | H | H | OH | H | H |
| I-13 | O | H | H | FX2 | H | H | H | H | H | H |
| I-14 | O | H | H | FX2 | H | H | H | NH₂ | H | H |
| I-15 | O | H | H | FX2 | H | H | H | OMe | H | H |
| I-16 | O | H | H | FX2 | H | H | H | OH | H | H |
| I-17 | O | H | H | FX3 | H | H | H | H | H | H |
| I-18 | O | H | H | FX3 | H | H | H | NH₂ | H | H |
| I-19 | O | H | H | FX3 | H | H | H | OMe | H | H |
| I-20 | O | H | H | FX3 | H | H | H | OH | H | H |
| I-21 | O | H | H | H | H | H | H | F | H | H |
| I-22 | O | H | H | H | H | H | H | I | H | H |
| I-23 | O | H | H | H | H | H | H | FX | H | H |
| I-24 | O | H | H | H | H | H | H | FX2 | H | H |
| I-25 | O | H | H | H | H | H | H | FX3 | H | H |
| I-26 | O | H | H | H | H | NMe₂ | H | H | H | H |
| I-27 | O | H | H | H | H | H | NMe₂ | H | H | H |
| I-28 | O | H | H | H | H | H | H | NMe₂ | H | H |
| I-29 | O | H | H | H | H | H | H | H | NMe₂ | H |
| I-30 | O | H | H | H | H | H | H | H | H | NMe₂ |
| I-31 | O | H | H | H | H | NHMe | H | H | H | H |
| I-32 | O | H | H | H | H | H | NHMe | H | H | H |
| I-33 | O | H | H | H | H | H | H | NHMe | H | H |
| I-34 | O | H | H | H | H | H | H | H | NHMe | H |
| I-35 | O | H | H | H | H | H | H | H | H | NHMe |
| I-36 | O | F | H | H | H | NMe₂ | H | H | H | H |
| I-37 | O | H | F | H | H | NMe₂ | H | H | H | H |
| I-38 | O | H | H | F | H | NMe₂ | H | H | H | H |
| I-39 | O | H | H | H | F | NMe₂ | H | H | H | H |
| I-90 | O | F | H | H | H | H | NMe₂ | H | H | H |
| I-41 | O | H | F | H | H | H | NMe₂ | H | H | H |
| I-42 | O | H | H | F | H | H | NMe₂ | H | H | H |
| I-43 | O | H | H | H | F | H | NMe₂ | H | H | H |
| I-44 | O | F | H | H | H | H | H | NMe₂ | H | H |
| I-45 | O | H | F | H | H | H | H | NMe₂ | H | H |
| I-46 | O | H | H | F | H | H | H | NMe₂ | H | H |
| I-47 | O | H | H | H | F | H | H | NMe₂ | H | H |
| I-48 | O | F | H | H | H | H | H | H | NMe₂ | H |
| I-49 | O | H | F | H | H | H | H | H | NMe₂ | H |
| I-50 | O | H | H | F | H | H | H | H | NMe₂ | H |
| I-51 | O | H | H | H | F | H | H | H | NMe₂ | H |
| I-52 | O | F | H | H | H | H | H | H | H | NMe₂ |
| I-53 | O | H | F | H | H | H | H | H | H | NMe₂ |
| I-54 | O | H | H | F | H | H | H | H | H | NMe₂ |
| I-55 | O | H | H | H | F | H | H | H | H | NMe₂ |
| I-56 | O | F | H | H | H | NHMe | H | H | H | H |
| I-57 | O | H | F | H | H | NHMe | H | H | H | H |
| I-58 | O | H | H | F | H | NHMe | H | H | H | H |
| I-59 | O | H | H | H | F | NHMe | H | H | H | H |
| I-60 | O | F | H | H | H | H | NHMe | H | H | H |
| I-61 | O | H | F | H | H | H | NHMe | H | H | H |
| I-62 | O | H | H | F | H | H | NHMe | H | H | H |
| I-63 | O | H | H | H | F | H | NHMe | H | H | H |
| I-64 | O | F | H | H | H | H | H | NHMe | H | H |
| I-65 | O | H | F | H | H | H | H | NHMe | H | H |
| I-66 | O | H | H | F | H | H | H | NHMe | H | H |

**Table 2**

| | | | | | | | | | | |
|---|---|---|---|---|---|---|---|---|---|---|
| I-67 | O | H | H | H | F | H | H | NHMe | H | H |
| I-68 | O | F | H | H | H | H | H | H | NHMe | H |
| 1-69 | O | H | F | H | H | H | H | H | NHMe | H |
| I-70 | O | H | H | F | H | H | H | H | NHMe | H |
| I-71 | O | H | H | H | F | H | H | H | NHMe | H |
| I-72 | O | F | H | H | H | H | H | H | H | NHMe |
| I-73 | O | H | F | H | H | H | H | H | H | NHMe |
| I-74 | O | H | H | F | H | H | H | H | H | NHMe |
| I-75 | O | H | H | H | F | H | H | H | H | NHMe |
| I-76 | O | I | H | H | H | NMe₂ | H | H | H | H |
| I-77 | O | H | I | H | H | NMe₂ | H | H | H | H |
| I-78 | O | H | H | I | H | NMe₂ | H | H | H | H |
| I-79 | O | H | H | H | I | NMe₂ | H | H | H | H |
| I-80 | O | I | H | H | H | H | NMe₂ | H | H | H |
| I-81 | O | H | I | H | H | H | NMe₂ | H | H | H |
| I-82 | O | H | H | I | H | H | NMe₂ | H | H | H |
| I-83 | O | H | H | H | I | H | NMe₂ | H | H | H |
| I-84 | O | I | H | H | H | H | H | NMe₂ | H | H |
| I-85 | O | H | I | H | H | H | H | NMe₂ | H | H |
| I-86 | O | H | H | I | H | H | H | NMe₂ | H | H |
| I-87 | O | H | H | H | I | H | H | NMe₂ | H | H |
| I-88 | O | I | H | H | H | H | H | H | NMe₂ | H |
| I-89 | O | H | I | H | H | H | H | H | NMe₂ | H |
| I-90 | O | H | H | I | H | H | H | H | NMe₂ | H |
| I-91 | O | H | H | H | I | H | H | H | NMe₂ | H |
| I-92 | O | I | H | H | H | H | H | H | H | NMe₂ |
| I-93 | O | H | I | H | H | H | H | H | H | NMe₂ |
| I-94 | O | H | H | I | H | H | H | H | H | NMe₂ |
| I-95 | O | H | H | H | I | H | H | H | H | NMe₂ |
| I-96 | O | I | H | H | H | NHMe | H | H | H | H |
| I-97 | O | H | I | H | H | NHMe | H | H | H | H |
| I-98 | O | H | H | I | H | NHMe | H | H | H | H |
| I-99 | O | H | H | H | I | NHMe | H | H | H | H |
| I-100 | O | I | H | H | H | H | NHMe | H | H | H |
| I-101 | O | H | I | H | H | H | NHMe | H | H | H |
| I-102 | O | H | H | I | H | H | NHMe | H | H | H |
| I-103 | O | H | H | H | I | H | NHMe | H | H | H |
| I-104 | O | I | H | H | H | H | H | NHMe | H | H |
| I-105 | O | H | I | H | H | H | H | NHMe | H | H |
| I-106 | O | H | H | I | H | H | H | NHMe | H | H |
| I-107 | O | H | H | H | I | H | H | NHMe | H | H |
| I-108 | O | I | H | H | H | H | H | H | NHMe | H |
| I-109 | O | H | I | H | H | H | H | H | NHMe | H |
| I-110 | O | H | H | I | H | H | H | H | NHMe | H |
| I-111 | O | H | H | H | I | H | H | H | NHMe | H |
| I-112 | O | I | H | H | H | H | H | H | H | NHMe |
| I-113 | O | H | I | H | H | H | H | H | H | NHMe |
| I-114 | O | H | H | I | H | H | H | H | H | NHMe |
| I-115 | O | H | H | H | I | H | H | H | H | NHMe |
| I-116 | O | FX | H | H | H | NMe₂ | H | H | H | H |
| I-117 | O | H | FX | H | H | NMe₂ | H | H | H | H |
| I-118 | O | H | H | FX | H | NMe₂ | H | H | H | H |
| I-119 | O | H | H | H | FX | NMe₂ | H | H | H | H |
| I-120 | O | FX | H | H | H | H | NMe₂ | H | H | H |
| I-121 | O | H | FX | H | H | H | NMe₂ | H | H | H |
| I-122 | O | H | H | FX | H | H | NMe₂ | H | H | H |
| I-123 | O | H | H | H | FX | H | NMe₂ | H | H | H |
| I-124 | O | FX | H | H | H | H | H | NMe₂ | H | H |
| I-125 | O | H | FX | H | H | H | H | NMe₂ | H | H |
| I-126 | O | H | H | FX | H | H | H | NMe₂ | H | H |
| I-127 | O | H | H | H | FX | H | H | NMe₂ | H | H |
| I-128 | O | FX | H | H | H | H | H | H | NMe₂ | H |
| I-129 | O | H | FX | H | H | H | H | H | NMe₂ | H |
| I-130 | O | H | H | FX | H | H | H | H | NMe₂ | H |
| I-131 | O | H | H | H | FX | H | H | H | NMe₂ | H |
| I-132 | O | FX | H | H | H | H | H | H | H | NMe₂ |
| I-133 | O | H | FX | H | H | H | H | H | H | NMe₂ |

**Table 3**

| | | | | | | | | | | |
|---|---|---|---|---|---|---|---|---|---|---|
| I-134 | O | H | H | FX | H | H | H | H | H | NMe₂ |
| I-135 | O | H | H | H | FX | H | H | H | H | NMe₂ |
| I-136 | O | FX | H | H | H | NHMe | H | H | H | H |
| I-137 | O | H | FX | H | H | NHMe | H | H | H | H |
| I-138 | O | H | H | FX | H | NHMe | H | H | H | H |
| I-139 | O | H | H | H | FX | NHMe | H | H | H | H |
| I-140 | O | FX | H | H | H | H | NHMe | H | H | H |
| I-141 | O | H | FX | H | H | H | NHMe | H | H | H |
| I-142 | O | H | H | FX | H | H | NHMe | H | H | H |
| I-143 | O | H | H | H | FX | H | NHMe | H | H | H |
| I-144 | O | FX | H | H | H | H | H | NHMe | H | H |
| I-145 | O | H | FX | H | H | H | H | NHMe | H | H |
| I-196 | O | H | H | FX | H | H | H | NHMe | H | H |
| I-197 | O | H | H | H | FX | H | H | NHMe | H | H |
| I-148 | O | FX | H | H | H | H | H | H | NHMe | H |
| I-149 | O | H | FX | H | H | H | H | H | NHMe | H |
| I-150 | O | H | H | FX | H | H | H | H | NHMe | H |
| I-151 | O | H | H | H | FX | H | H | H | NHMe | H |
| I-152 | O | FX | H | H | H | H | H | H | H | NHMe |
| I-153 | O | H | FX | H | H | H | H | H | H | NHMe |
| I-154 | O | H | H | FX | H | H | H | H | H | NHMe |
| I-155 | O | H | H | H | FX | H | H | H | H | NHMe |
| I-156 | O | FX2 | H | H | H | NMe₂ | H | H | H | H |
| I-157 | O | H | FX2 | H | H | NMe₂ | H | H | H | H |
| I-158 | O | H | H | FX2 | H | NMe₂ | H | H | H | H |
| I-159 | O | H | H | H | FX2 | NMe₂ | H | H | H | H |
| I-160 | O | FX2 | H | H | H | H | NMe₂ | H | H | H |
| I-161 | O | H | FX2 | H | H | H | NMe₂ | H | H | H |
| I-162 | O | H | H | FX2 | H | H | NMe₂ | H | H | H |
| I-163 | O | H | H | H | FX2 | H | NMe₂ | H | H | H |
| I-164 | O | FX2 | H | H | H | H | H | NMe₂ | H | H |
| I-165 | O | H | FX2 | H | H | H | H | NMe₂ | H | H |
| I-166 | O | H | H | FX2 | H | H | H | NMe₂ | H | H |
| I-167 | O | H | H | H | FX2 | H | H | NMe_{z} | H | H |
| I-168 | O | FX2 | H | H | H | H | H | H | NMe₂ | H |
| I-169 | O | H | FX2 | H | H | H | H | H | NMe₂ | H |
| I-170 | O | H | H | FX2 | H | H | H | H | NMe₂ | H |
| I-171 | O | H | H | H | FX2 | H | H | H | NMe₂ | H |
| I-172 | O | FX2 | H | H | H | H | H | H | H | NMe₂ |
| I-173 | O | H | FX2 | H | H | H | H | H | H | NMe₂ |
| I-174 | O | H | H | FX2 | H | H | H | H | H | NMe₂ |
| I-175 | O | H | H | H | FX2 | H | H | H | H | NMe₂ |
| I-176 | O | FX2 | H | H | H | NHMe | H | H | H | H |
| I-177 | O | H | FX2 | H | H | NHMe | H | H | H | H |
| I-178 | O | H | H | FX2 | H | NHMe | H | H | H | H |
| I-179 | O | H | H | H | FX2 | NHMe | H | H | H | H |
| I-180 | O | FX2 | H | H | H | H | NHMe | H | H | H |
| I-181 | O | H | FX2 | H | H | H | NHMe | H | H | H |
| I-182 | O | H | H | FX2 | H | H | NHMe | H | H | H |
| I-183 | O | H | H | H | FX2 | H | NHMe | H | H | H |
| I-184 | O | FX2 | H | H | H | H | H | NHMe | H | H |
| I-185 | O | H | FX2 | H | H | H | H | NHMe | H | H |
| I-186 | O | H | H | FX2 | H | H | H | NHMe | H | H |
| I-187 | O | H | H | H | FX2 | H | H | NHMe | H | H |
| I-188 | O | FX2 | H | H | H | H | H | H | NHMe | H |
| I-189 | O | H | FX2 | H | H | H | H | H | NHMe | H |
| I-190 | O | H | H | FX2 | H | H | H | H | NHMe | H |
| I-191 | O | H | H | H | FX2 | H | H | H | NHMe | H |
| I-192 | O | FX2 | H | H | H | H | H | H | H | NHMe |
| I-193 | O | H | FX2 | H | H | H | H | H | H | NHMe |
| I-194 | O | H | H | FX2 | H | H | H | H | H | NHMe |
| I-195 | O | H | H | H | FX2 | H | H | H | H | NHMe |
| I-196 | O | FX3 | H | H | H | NMe₂ | H | H | H | H |
| I-197 | O | H | FX3 | H | H | NMe₂ | H | H | H | H |
| I-198 | O | H | H | FX3 | H | NMe₂ | H | H | H | H |
| I-199 | O | H | H | H | FX3 | NMe₂ | H | H | H | H |
| I-200 | O | FX3 | H | H | H | H | NMe₂ | H | H | H |

**Table 4**

| | | | | | | | | | | |
|---|---|---|---|---|---|---|---|---|---|---|
| I-201 | O | H | FX3 | H | H | H | NMe₂ | H | H | H |
| I-202 | O | H | H | FX3 | H | H | NMe₂ | H | H | H |
| I-203 | O | H | H | H | FX3 | H | NMe₂ | H | H | H |
| I-204 | O | FX3 | H | H | H | H | H | NMe₂ | H | H |
| I-205 | O | H | FX3 | H | H | H | H | NMe₂ | H | H |
| I-206 | O | H | H | FX3 | H | H | H | NMe₂ | H | H |
| I-207 | O | H | H | H | FX3 | H | H | NMe₂ | H | H |
| I-208 | O | FX3 | H | H | H | H | H | H | NMe₂ | H |
| I-209 | O | H | FX3 | H | H | H | H | H | NMe₂ | H |
| I-210 | O | H | H | FX3 | H | H | H | H | NMe₂ | H |
| I-211 | O | H | H | H | FX3 | H | H | H | NMe₂ | H |
| I-212 | O | FX3 | H | H | H | H | H | H | H | NMe₂ |
| I-213 | O | H | FX3 | H | H | H | H | H | H | NMe₂ |
| I-214 | O | H | H | FX3 | H | H | H | H | H | NMe₂ |
| I-215 | O | H | H | H | FX3 | H | H | H | H | NMe₂ |
| I-216 | O | FX3 | H | H | H | NHMe | H | H | H | H |
| I-217 | O | H | FX3 | H | H | NHMe | H | H | H | H |
| I-218 | O | H | H | FX3 | H | NHMe | H | H | H | H |
| I-219 | O | H | H | H | FX3 | NHMe | H | H | H | H |
| I-220 | O | FX3 | H | H | H | H | NHMe | H | H | H |
| I-221 | O | H | FX3 | H | H | H | NHMe | H | H | H |
| I-222 | O | H | H | FX3 | H | H | NHMe | H | H | H |
| I-223 | O | H | H | H | FX3 | H | NHMe | H | H | H |
| I-224 | O | FX3 | H | H | H | H | H | NHMe | H | H |
| I-225 | O | H | FX3 | H | H | H | H | NHMe | H | H |
| I-226 | O | H | H | FX3 | H | H | H | NHMe | H | H |
| I-227 | O | H | H | H | FX3 | H | H | NHMe | H | H |
| I-228 | O | FX3 | H | H | H | H | H | H | NHMe | H |
| I-229 | O | H | FX3 | H | H | H | H | H | NHMe | H |
| I-230 | O | H | H | FX3 | H | H | H | H | NHMe | H |
| I-231 | O | H | H | H | FX3 | H | H | H | NHMe | H |
| I-232 | O | FX3 | H | H | H | H | H | H | H | NHMe |
| I-233 | O | H | FX3 | H | H | H | H | H | H | NHMe |
| I-234 | O | H | H | FX3 | H | H | H | H | H | NHMe |
| I-235 | O | H | H | H | FX3 | H | H | H | H | NHMe |
| I-236 | NH | H | H | F | H | H | H | H | H | H |
| I-237 | NH | H | H | F | H | H | H | NH₂ | H | H |
| I-238 | NH | H | H | F | H | H | H | OMe | H | H |
| I-239 | NH | H | H | F | H | H | H | OH | H | H |
| I-240 | NH | H | H | I | H | H | H | H | H | H |
| 1-241 | NH | H | H | I | H | H | H | NH₂ | H | H |
| I-242 | NH | H | H | I | H | H | H | OMe | H | H |
| I-243 | NH | H | H | I | H | H | H | OH | H | H |
| I-244 | NH | H | H | FX | H | H | H | H | H | H |
| I-245 | NH | H | H | FX | H | H | H | NH₂ | H | H |
| I-246 | NH | H | H | FX | H | H | H | OMe | H | H |
| I-247 | NH | H | H | FX | H | H | H | OH | H | H |
| I-248 | NH | H | H | FX2 | H | H | H | H | H | H |
| I-249 | NH | H | H | FX2 | H | H | H | NH₂ | H | H |
| I-250 | NH | H | H | FX2 | H | H | H | OMe | H | H |
| 1-251 | NH | H | H | FX2 | H | H | H | OH | H | H |
| I-252 | NH | H | H | FX3 | H | H | H | H | H | H |
| I-253 | NH | H | H | FX3 | H | H | H | NH₂ | H | H |
| I-254 | NH | H | H | FX3 | H | H | H | OMe | H | H |
| I-255 | NH | H | H | FX3 | H | H | H | OH | H | H |
| I-256 | NH | H | H | H | H | H | H | F | H | H |
| I-257 | NH | H | H | H | H | H | H | I | H | H |
| I-258 | NH | H | H | H | H | H | H | FX | H | H |
| I-259 | NH | H | H | H | H | H | H | FX2 | H | H |
| I-260 | NH | H | H | H | H | H | H | FX3 | H | H |
| I-261 | NH | H | H | H | H | NMe₂ | H | H | H | H |
| I-262 | NH | H | H | H | H | H | NMe₂ | H | H | H |
| I-263 | NH | H | H | H | H | H | H | NMe₂ | H | H |
| I-264 | NH | H | H | H | H | H | H | H | NMe₂ | H |
| I-265 | NH | H | H | H | H | H | H | H | H | NMe₂ |
| I-266 | NH | H | H | H | H | NHMe | H | H | H | H |
| I-267 | NH | H | H | H | H | H | NHMe | H | H | H |

**Table 5**

| | | | | | | | | | | |
|---|---|---|---|---|---|---|---|---|---|---|
| I-268 | NH | H | H | H | H | H | H | NHMe | H | H |
| I-269 | NH | H | H | H | H | H | H | H | NHMe | H |
| I-270 | NH | H | H | H | H | H | H | H | H | NHMe |
| I-271 | NH | F | H | H | H | NMe₂ | H | H | H | H |
| I-272 | NH | H | F | H | H | NMe₂ | H | H | H | H |
| I-273 | NH | H | H | F | H | NMe₂ | H | H | H | H |
| I-274 | NH | H | H | H | F | NMe₂ | H | H | H | H |
| I-275 | NH | F | H | H | H | H | NMe₂ | H | H | H |
| I-276 | NH | H | F | H | H | H | NMe₂ | H | H | H |
| I-277 | NH | H | H | F | H | H | NMe₂ | H | H | H |
| I-278 | NH | H | H | H | F | H | NMe₂ | H | H | H |
| I-279 | NH | F | H | H | H | H | H | NMe₂ | H | H |
| I-280 | NH | H | F | H | H | H | H | NMe₂ | H | H |
| I-281 | NH | H | H | F | H | H | H | NMe₂ | H | H |
| I-282 | NH | H | H | H | F | H | H | NMe₂ | H | H |
| I-283 | NH | F | H | H | H | H | H | H | NMe₂ | H |
| I-284 | NH | H | F | H | H | H | H | H | NMe₂ | H |
| I-285 | NH | H | H | F | H | H | H | H | NMe₂ | H |
| I-286 | NH | H | H | H | F | H | H | H | NMe₂ | H |
| I-287 | NH | F | H | H | H | H | H | H | H | NMe₂ |
| I-288 | NH | H | F | H | H | H | H | H | H | NMe₂ |
| I-289 | NH | H | H | F | H | H | H | H | H | NMe₂ |
| I-290 | NH | H | H | H | F | H | H | H | H | NMe₂ |
| I-291 | NH | F | H | H | H | NHMe | H | H | H | H |
| I-292 | NH | H | F | H | H | NHMe | H | H | H | H |
| I-293 | NH | H | H | F | H | NHMe | H | H | H | H |
| I-294 | NH | H | H | H | F | NHMe | H | H | H | H |
| I-295 | NH | F | H | H | H | H | NHMe | H | H | H |
| I-296 | NH | H | F | H | H | H | NHMe | H | H | H |
| I-297 | NH | H | H | F | H | H | NHMe | H | H | H |
| I-298 | NH | H | H | H | F | H | NHMe | H | H | H |
| I-299 | NH | F | H | H | H | H | H | NHMe | H | H |
| I-300 | NH | H | F | H | H | H | H | NHMe | H | H |
| I-301 | NH | H | H | F | H | H | H | NHMe | H | H |
| I-302 | NH | H | H | H | F | H | H | NHMe | H | H |
| I-303 | NH | F | H | H | H | H | H | H | NHMe | H |
| I-304 | NH | H | F | H | H | H | H | H | NHMe | H |
| I-305 | NH | H | H | F | H | H | H | H | NHMe | H |
| I-306 | NH | H | H | H | F | H | H | H | NHMe | H |
| I-307 | NH | F | H | H | H | H | H | H | H | NHMe |
| I-308 | NH | H | F | H | H | H | H | H | H | NHMe |
| I-309 | NH | H | H | F | H | H | H | H | H | NHMe |
| I-310 | NH | H | H | H | F | H | H | H | H | NHMe |
| I-311 | NH | I | H | H | H | NMe₂ | H | H | H | H |
| I-312 | NH | H | I | H | H | NMe₂ | H | H | H | H |
| I-313 | NH | H | H | I | H | NMe₂ | H | H | H | H |
| I-314 | NH | H | H | H | I | NMe₂ | H | H | H | H |
| I-315 | NH | I | H | H | H | H | NMe₂ | H | H | H |
| I-316 | NH | H | I | H | H | H | NMe₂ | H | H | H |
| I-317 | NH | H | H | I | H | H | NMe₂ | H | H | H |
| I-318 | NH | H | H | H | I | H | NMe₂ | H | H | H |
| I-319 | NH | I | H | H | H | H | H | NMe₂ | H | H |
| I-320 | NH | H | I | H | H | H | H | NMe₂ | H | H |
| I-321 | NH | H | H | I | H | H | H | NMe₂ | H | H |
| I-322 | NH | H | H | H | I | H | H | NMe₂ | H | H |
| I-323 | NH | I | H | H | H | H | H | H | NMe₂ | H |
| I-324 | NH | H | I | H | H | H | H | H | NMe₂ | H |
| I-325 | NH | H | H | I | H | H | H | H | NMe₂ | H |
| I-326 | NH | H | H | H | I | H | H | H | NMe₂ | H |
| I-327 | NH | I | H | H | H | H | H | H | H | NMe₂ |
| I-328 | NH | H | I | H | H | H | H | H | H | NMe₂ |
| I-329 | NH | H | H | I | H | H | H | H | H | NMe₂ |
| I-330 | NH | H | H | H | I | H | H | H | H | NMe₂ |
| I-331 | NH | I | H | H | H | NHMe | H | H | H | H |
| I-332 | NH | H | I | H | H | NHMe | H | H | H | H |
| I-333 | NH | H | H | I | H | NHMe | H | H | H | H |
| I-334 | NH | H | H | H | I | NHMe | H | H | H | H |

**Table 6**

| | | | | | | | | | | |
|---|---|---|---|---|---|---|---|---|---|---|
| I-335 | NH | I | H | H | H | H | NHMe | H | H | H |
| I-336 | NH | H | I | H | H | H | NHMe | H | H | H |
| I-337 | NH | H | H | I | H | H | NHMe | H | H | H |
| I-338 | NH | H | H | H | I | H | NHMe | H | H | H |
| I-339 | NH | I | H | H | H | H | H | NHMe | H | H |
| I-340 | NH | H | I | H | H | H | H | NHMe | H | H |
| I-341 | NH | H | H | I | H | H | H | NHMe | H | H |
| I-342 | NH | H | H | H | I | H | H | NHMe | H | H |
| I-343 | NH | I | H | H | H | H | H | H | NHMe | H |
| I-344 | NH | H | I | H | H | H | H | H | NHMe | H |
| I-345 | NH | H | H | I | H | H | H | H | NHMe | H |
| I-346 | NH | H | H | H | I | H | H | H | NHMe | H |
| I-347 | NH | I | H | H | H | H | H | H | H | NHMe |
| I-348 | NH | H | I | H | H | H | H | H | H | NHMe |
| I-349 | NH | H | H | I | H | H | H | H | H | NHMe |
| I-350 | NH | H | H | H | I | H | H | H | H | NHMe |
| I-351 | NH | FX | H | H | H | NMe₂ | H | H | H | H |
| I-352 | NH | H | FX | H | H | NMe₂ | H | H | H | H |
| I-353 | NH | H | H | FX | H | NMe₂ | H | H | H | H |
| I-359 | NH | H | H | H | FX | NMe₂ | H | H | H | H |
| I-355 | NH | FX | H | H | H | H | NMe₂ | H | H | H |
| I-356 | NH | H | FX | H | H | H | NMe₂ | H | H | H |
| I-357 | NH | H | H | FX | H | H | NMe₂ | H | H | H |
| I-358 | NH | H | H | H | FX | H | NMe₂ | H | H | H |
| I-359 | NH | FX | H | H | H | H | H | NMe₂ | H | H |
| I-360 | NH | H | FX | H | H | H | H | NMe₂ | H | H |
| I-361 | NH | H | H | FX | H | H | H | NMe₂ | H | H |
| I-362 | NH | H | H | H | FX | H | H | NMe₂ | H | H |
| I-363 | NH | FX | H | H | H | H | H | H | NMe₂ | H |
| I-364 | NH | H | FX | H | H | H | H | H | NMe₂ | H |
| I-365 | NH | H | H | FX | H | H | H | H | NMe₂ | H |
| I-366 | NH | H | H | H | FX | H | H | H | NMe₂ | H |
| I-367 | NH | FX | H | H | H | H | H | H | H | NMe₂ |
| I-368 | NH | H | FX | H | H | H | H | H | H | NMe₂ |
| I-369 | NH | H | H | FX | H | H | H | H | H | NMe₂ |
| I-370 | NH | H | H | H | FX | H | H | H | H | NMe₂ |
| I-371 | NH | FX | H | H | H | NHMe | H | H | H | H |
| I-372 | NH | H | FX | H | H | NHMe | H | H | H | H |
| I-373 | NH | H | H | FX | H | NHMe | H | H | H | H |
| I-374 | NH | H | H | H | FX | NHMe | H | H | H | H |
| I-375 | NH | FX | H | H | H | H | NHMe | H | H | H |
| I-376 | NH | H | FX | H | H | H | NHMe | H | H | H |
| I-377 | NH | H | H | FX | H | H | NHMe | H | H | H |
| I-378 | NH | H | H | H | FX | H | NHMe | H | H | H |
| I-379 | NH | FX | H | H | H | H | H | NHMe | H | H |
| I-380 | NH | H | FX | H | H | H | H | NHMe | H | H |
| I-381 | NH | H | H | FX | H | H | H | NHMe | H | H |
| I-382 | NH | H | H | H | FX | H | H | NHMe | H | H |
| I-383 | NH | FX | H | H | H | H | H | H | NHMe | H |
| I-384 | NH | H | FX | H | H | H | H | H | NHMe | H |
| I-385 | NH | H | H | FX | H | H | H | H | NHMe | H |
| I-386 | NH | H | H | H | FX | H | H | H | NHMe | H |
| I-387 | NH | FX | H | H | H | H | H | H | H | NHMe |
| I-388 | NH | H | FX | H | H | H | H | H | H | NHMe |
| I-389 | NH | H | H | FX | H | H | H | H | H | NHMe |
| I-390 | NH | H | H | H | FX | H | H | H | H | NHMe |
| I-391 | NH | FX2 | H | H | H | NMe₂ | H | H | H | H |
| I-392 | NH | H | FX2 | H | H | NMe₂ | H | H | H | H |
| I-393 | NH | H | H | FX2 | H | NMe₂ | H | H | H | H |
| I-394 | NH | H | H | H | FX2 | NMe₂ | H | H | H | H |
| I-395 | NH | FX2 | H | H | H | H | NMe₂ | H | H | H |
| I-396 | NH | H | FX2 | H | H | H | NMe₂ | H | H | H |
| I-397 | NH | H | H | FX2 | H | H | NMe₂ | H | H | H |
| I-398 | NH | H | H | H | FX2 | H | NMe₂ | H | H | H |
| I-399 | NH | FX2 | H | H | H | H | H | NMe₂ | H | H |
| I-400 | NH | H | FX2 | H | H | H | H | NMe₂ | H | H |
| I-401 | NH | H | H | FX2 | H | H | H | NMe₂ | H | H |

**Table 7**

| | | | | | | | | | | |
|---|---|---|---|---|---|---|---|---|---|---|
| I-402 | NH | H | H | H | FX2 | H | H | NMe₂ | H | H |
| I-403 | NH | FX2 | H | H | H | H | H | H | NMe₂ | H |
| I-404 | NH | H | FX2 | H | H | H | H | H | NMe₂ | H |
| I-405 | NH | H | H | FX2 | H | H | H | H | NMe₂ | H |
| I-406 | NH | H | H | H | FX2 | H | H | H | NMe₂ | H |
| I-407 | NH | FX2 | H | H | H | H | H | H | H | NMe₂ |
| I-408 | NH | H | FX2 | H | H | H | H | H | H | NMe₂ |
| I-409 | NH | H | H | FX2 | H | H | H | H | H | NMe₂ |
| I-410 | NH | H | H | H | FX2 | H | H | H | H | NMe₂ |
| I-411 | NH | FX2 | H | H | H | NHMe | H | H | H | H |
| I-412 | NH | H | FX2 | H | H | NHMe | H | H | H | H |
| I-413 | NH | H | H | FX2 | H | NHMe | H | H | H | H |
| I-414 | NH | H | H | H | FX2 | NHMe | H | H | H | H |
| I-415 | NH | FX2 | H | H | H | H | NHMe | H | H | H |
| I-416 | NH | H | FX2 | H | H | H | NHMe | H | H | H |
| I-417 | NH | H | H | FX2 | H | H | NHMe | H | H | H |
| I-418 | NH | H | H | H | FX2 | H | NHMe | H | H | H |
| I-419 | NH | FX2 | H | H | H | H | H | NHMe | H | H |
| I-420 | NH | H | FX2 | H | H | H | H | NHMe | H | H |
| I-421 | NH | H | H | FX2 | H | H | H | NHMe | H | H |
| I-422 | NH | H | H | H | FX2 | H | H | NHMe | H | H |
| I-423 | NH | FX2 | H | H | H | H | H | H | NHMe | H |
| I-424 | NH | H | FX2 | H | H | H | H | H | NHMe | H |
| I-425 | NH | H | H | FX2 | H | H | H | H | NHMe | H |
| I-426 | NH | H | H | H | FX2 | H | H | H | NHMe | H |
| I-427 | NH | FX2 | H | H | H | H | H | H | H | NHMe |
| I-428 | NH | H | FX2 | H | H | H | H | H | H | NHMe |
| I-429 | NH | H | H | FX2 | H | H | H | H | H | NHMe |
| I-430 | NH | H | H | H | FX2 | H | H | H | H | NHMe |
| I-431 | NH | FX3 | H | H | H | NMe₂ | H | H | H | H |
| I-432 | NH | H | FX3 | H | H | NMe₂ | H | H | H | H |
| I-433 | NH | H | H | FX3 | H | NMe₂ | H | H | H | H |
| I-434 | NH | H | H | H | FX3 | NMe₂ | H | H | H | H |
| I-435 | NH | FX3 | H | H | H | H | NMe₂ | H | H | H |
| I-436 | NH | H | FX3 | H | H | H | NMe₂ | H | H | H |
| I-437 | NH | H | H | FX3 | H | H | NMe₂ | H | H | H |
| I-438 | NH | H | H | H | FX3 | H | NMe₂ | H | H | H |
| I-439 | NH | FX3 | H | H | H | H | H | NMe₂ | H | H |
| I-440 | NH | H | FX3 | H | H | H | H | NMe₂ | H | H |
| I-441 | NH | H | H | FX3 | H | H | H | NMe₂ | H | H |
| I-442 | NH | H | H | H | FX3 | H | H | NMe₂ | H | H |
| I-443 | NH | FX3 | H | H | H | H | H | H | NMe₂ | H |
| I-444 | NH | H | FX3 | H | H | H | H | H | NMe₂ | H |
| I-445 | NH | H | H | FX3 | H | H | H | H | NMe₂ | H |
| I-446 | NH | H | H | H | FX3 | H | H | H | NMe₂ | H |
| I-447 | NH | FX3 | H | H | H | H | H | H | H | NMe₂ |
| I-448 | NH | H | FX3 | H | H | H | H | H | H | NMe₂ |
| I-449 | NH | H | H | FX3 | H | H | H | H | H | NMe₂ |
| I-450 | NH | H | H | H | FX3 | H | H | H | H | NMe₂ |
| I-451 | NH | FX3 | H | H | H | NHMe | H | H | H | H |
| I-452 | NH | H | FX3 | H | H | NHMe | H | H | H | H |
| I-453 | NH | H | H | FX3 | H | NHMe | H | H | H | H |
| I-454 | NH | H | H | H | FX3 | NHMe | H | H | H | H |
| I-455 | NH | FX3 | H | H | H | H | NHMe | H | H | H |
| I-456 | NH | H | FX3 | H | H | H | NHMe | H | H | H |
| I-457 | NH | H | H | FX3 | H | H | NHMe | H | H | H |
| I-458 | NH | H | H | H | FX3 | H | NHMe | H | H | H |
| I-459 | NH | FX3 | H | H | H | H | H | NHMe | H | H |
| I-460 | NH | H | FX3 | H | H | H | H | NHMe | H | H |
| I-461 | NH | H | H | FX3 | H | H | H | NHMe | H | H |
| I-462 | NH | H | H | H | FX3 | H | H | NHMe | H | H |
| I-463 | NH | FX3 | H | H | H | H | H | H | NHMe | H |
| I-464 | NH | H | FX3 | H | H | H | H | H | NHMe | H |
| I-465 | NH | H | H | FX3 | H | H | H | H | NHMe | H |
| I-466 | NH | H | H | H | FX3 | H | H | H | NHMe | H |
| I-467 | NH | FX3 | H | H | H | H | H | H | H | NHMe |
| I-468 | NH | H | FX3 | H | H | H | H | H | H | NHMe |

**Table 8**

| | | | | | | | | | | |
|---|---|---|---|---|---|---|---|---|---|---|
| I-469 | NH | H | H | FX3 | H | H | H | H | H | NHMe |
| I-470 | NH | H | H | H | FX3 | H | H | H | H | NHMe |
| I-471 | O | H | H | HOX | H | H | H | H | H | H |
| I-472 | O | H | H | HOX | H | H | H | NH₂ | H | H |
| I-473 | O | H | H | HOX | H | H | H | OMe | H | H |
| I-474 | O | H | H | HOX | H | H | H | OH | H | H |
| I-475 | O | H | H | HOX2 | H | H | H | H | H | H |
| I-476 | O | H | H | HOX2 | H | H | H | NH₂ | H | H |
| I-477 | O | H | H | HOX2 | H | H | H | OMe | H | H |
| I-478 | O | H | H | HOX2 | H | H | H | OH | H | H |
| I-479 | O | H | H | HOX3 | H | H | H | H | H | H |
| I-480 | O | H | H | HOX3 | H | H | H | NH₂ | H | H |
| I-481 | O | H | H | HOX3 | H | H | H | OMe | H | H |
| I-482 | O | H | H | HOX3 | H | H | H | OH | H | H |
| I-483 | O | H | H | H | H | H | H | HOX | H | H |
| I-484 | O | H | H | H | H | H | H | HOX2 | H | H |
| I-485 | O | H | H | H | H | H | H | HOX3 | H | H |
| I-486 | O | HOX | H | H | H | NMe₂ | H | H | H | H |
| I-487 | O | H | HOX | H | H | NMe₂ | H | H | H | H |
| I-488 | O | H | H | HOX | H | NMe₂ | H | H | H | H |
| I-489 | O | H | H | H | HOX | NMe₂ | H | H | H | H |
| I-490 | O | HOX | H | H | H | H | NMe₂ | H | H | H |
| I-491 | O | H | HOX | H | H | H | NMe₂ | H | H | H |
| I-492 | O | H | H | HOX | H | H | NMe₂ | H | H | H |
| I-493 | O | H | H | H | HOX | H | NMe₂ | H | H | H |
| I-494 | O | HOX | H | H | H | H | H | NMe₂ | H | H |
| I-495 | O | H | HOX | H | H | H | H | NMe₂ | H | H |
| I-496 | O | H | H | HOX | H | H | H | NMe₂ | H | H |
| I-497 | O | H | H | H | HOX | H | H | NMe₂ | H | H |
| I-498 | O | HOX | H | H | H | H | H | H | NMe₂ | H |
| I-499 | O | H | HOX | H | H | H | H | H | NMe₂ | H |
| I-500 | O | H | H | HOX | H | H | H | H | NMe₂ | H |
| I-501 | O | H | H | H | HOX | H | H | H | NMe₂ | H |
| I-502 | O | HOX | H | H | H | H | H | H | H | NMe₂ |
| I-503 | O | H | HOX | H | H | H | H | H | H | NMe₂ |
| I-504 | O | H | H | HOX | H | H | H | H | H | NMe₂ |
| I-505 | O | H | H | H | HOX | H | H | H | H | NMe₂ |
| I-506 | O | HOX | H | H | H | NHMe | H | H | H | H |
| I-507 | O | H | HOX | H | H | NHMe | H | H | H | H |
| I-508 | O | H | H | HOX | H | NHMe | H | H | H | H |
| I-509 | O | H | H | H | HOX | NHMe | H | H | H | H |
| I-510 | O | HOX | H | H | H | H | NHMe | H | H | H |
| I-511 | O | H | HOX | H | H | H | NHMe | H | H | H |
| I-512 | O | H | H | HOX | H | H | NHMe | H | H | H |
| I-513 | O | H | H | H | HOX | H | NHMe | H | H | H |
| I-514 | O | HOX | H | H | H | H | H | NHMe | H | H |
| I-515 | O | H | HOX | H | H | H | H | NHMe | H | H |
| I-516 | O | H | H | HOX | H | H | H | NHMe | H | H |
| I-517 | O | H | H | H | HOX | H | H | NHMe | H | H |
| I-518 | O | HOX | H | H | H | H | H | H | NHMe | H |
| I-519 | O | H | HOX | H | H | H | H | H | NHMe | H |
| I-520 | O | H | H | HOX | H | H | H | H | NHMe | H |
| I-521 | O | H | H | H | HOX | H | H | H | NHMe | H |
| I-522 | O | HOX | H | H | H | H | H | H | H | NHMe |
| I-523 | O | H | HOX | H | H | H | H | H | H | NHMe |
| I-524 | O | H | H | HOX | H | H | H | H | H | NHMe |
| I-525 | O | H | H | H | HOX | H | H | H | H | NHMe |
| I-526 | O | HOX2 | H | H | H | NMe₂ | H | H | H | H |
| I-527 | O | H | HOX2 | H | H | NMe₂ | H | H | H | H |
| I-528 | O | H | H | HOX2 | H | NMe₂ | H | H | H | H |
| I-529 | O | H | H | H | HOX2 | NMe₂ | H | H | H | H |
| I-530 | O | HOX2 | H | H | H | H | NMe₂ | H | H | H |
| I-531 | O | H | HOX2 | H | H | H | NMe₂ | H | H | H |
| I-532 | O | H | H | HOX2 | H | H | NMe₂ | H | H | H |
| I-533 | O | H | H | H | HOX2 | H | NMe₂ | H | H | H |
| I-534 | O | HOX2 | H | H | H | H | H | NMe₂ | H | H |
| I-535 | O | H | HOX2 | H | H | H | H | NMe₂ | H | H |

**Table 9**

| | | | | | | | | | | |
|---|---|---|---|---|---|---|---|---|---|---|
| I-536 | O | H | H | HOX2 | H | H | H | NMe₂ | H | H |
| I-537 | O | H | H | H | HOX2 | H | H | NMe₂ | H | H |
| I-538 | O | HOX2 | H | H | H | H | H | H | AMe₂ | H |
| I-539 | O | H | HOX2 | H | H | H | H | H | AMe₂ | H |
| I-540 | O | H | H | HOX2 | H | H | H | H | NMe₂ | H |
| I-541 | O | H | H | H | HOX2 | H | H | H | NMe₂ | H |
| I-542 | O | HOX2 | H | H | H | H | H | H | H | NMe₂ |
| I-543 | O | H | HOX2 | H | H | H | H | H | H | NMe₂ |
| I-544 | O | H | H | HOX2 | H | H | H | H | H | NMe₂ |
| I-545 | O | H | H | H | HOX2 | H | H | H | H | NMe₂ |
| I-546 | O | HOX2 | H | H | H | NHMe | H | H | H | H |
| I-547 | O | H | HOX2 | H | H | NHMe | H | H | H | H |
| I-548 | O | H | H | HOX2 | H | NHMe | H | H | H | H |
| I-549 | O | H | H | H | HOX2 | NHMe | H | H | H | H |
| I-550 | O | HOX2 | H | H | H | H | NHMe | H | H | H |
| I-551 | O | H | HOX2 | H | H | H | NHMe | H | H | H |
| I-552 | O | H | H | HOX2 | H | H | NHMe | H | H | H |
| I-553 | O | H | H | H | HOX2 | H | NHMe | H | H | H |
| I-554 | O | HOX2 | H | H | H | H | H | NHMe | H | H |
| I-555 | O | H | HOX2 | H | H | H | H | NHMe | H | H |
| I-556 | O | H | H | HOX2 | H | H | H | NHMe | H | H |
| I-557 | O | H | H | H | HOX2 | H | H | NHMe | H | H |
| I-558 | O | HOX2 | H | H | H | H | H | H | NHMe | H |
| I-559 | O | H | HOX2 | H | H | H | H | H | NHMe | H |
| I-560 | O | H | H | HOX2 | H | H | H | H | NHMe | H |
| I-561 | O | H | H | H | HOX2 | H | H | H | NHMe | H |
| I-562 | O | HOX2 | H | H | H | H | H | H | H | NHMe |
| I-563 | O | H | HOX2 | H | H | H | H | H | H | NHMe |
| I-564 | O | H | H | HOX2 | H | H | H | H | H | NHMe |
| I-565 | O | H | H | H | HOX2 | H | H | H | H | NHMe |
| I-566 | O | HOX3 | H | H | H | NMe₂ | H | H | H | H |
| I-567 | O | H | HOX3 | H | H | NMe₂ | H | H | H | H |
| I-568 | O | H | H | HOX3 | H | NMe₂ | H | H | H | H |
| I-569 | O | H | H | H | HOX3 | NMe₂ | H | H | H | H |
| I-570 | O | HOX3 | H | H | H | H | NMe₂ | H | H | H |
| I-571 | O | H | HOX3 | H | H | H | NMe₂ | H | H | H |
| I-572 | O | H | H | HOX3 | H | H | NMe₂ | H | H | H |
| I-573 | O | H | H | H | HOX3 | H | NMe₂ | H | H | H |
| I-574 | O | HOX3 | H | H | H | H | H | NMe₂ | H | H |
| I-575 | O | H | HOX3 | H | H | H | H | NMe₂ | H | H |
| I-576 | O | H | H | HOX3 | H | H | H | NMe₂ | H | H |
| I-577 | O | H | H | H | HOX3 | H | H | NMe₂ | H | H |
| I-578 | O | HOX3 | H | H | H | H | H | H | NMe₂ | H |
| I-579 | O | H | HOX3 | H | H | H | H | H | NMe₂ | H |
| I-580 | O | H | H | HOX3 | H | H | H | H | NMe₂ | H |
| I-581 | O | H | H | H | HOX3 | H | H | H | NMe₂ | H |
| I-582 | O | HOX3 | H | H | H | H | H | H | H | NMe₂ |
| I-583 | O | H | HOX3 | H | H | H | H | H | H | NMe₂ |
| I-584 | O | H | H | HOX3 | H | H | H | H | H | NMe₂ |
| I-585 | O | H | H | H | HOX3 | H | H | H | H | NMe₂ |
| I-586 | O | HOX3 | H | H | H | NHMe | H | H | H | H |
| I-587 | O | H | HOX3 | H | H | NHMe | H | H | H | H |
| I-588 | O | H | H | HOX3 | H | NHMe | H | H | H | H |
| I-589 | O | H | H | H | HOX3 | NHMe | H | H | H | H |
| I-590 | O | HOX3 | H | H | H | H | NHMe | H | H | H |
| I-591 | O | H | HOX3 | H | H | H | NHMe | H | H | H |
| I-592 | O | H | H | HOX3 | H | H | NHMe | H | H | H |
| I-593 | O | H | H | H | HOX3 | H | NHMe | H | H | H |
| I-594 | O | HOX3 | H | H | H | H | H | NHMe | H | H |
| I-595 | O | H | HOX3 | H | H | H | H | NHMe | H | H |
| I-596 | O | H | H | HOX3 | H | H | H | NHMe | H | H |
| I-597 | O | H | H | H | HOX3 | H | H | NHMe | H | H |
| I-598 | O | HOX3 | H | H | H | H | H | H | NHMe | H |
| I-599 | O | H | HOX3 | H | H | H | H | H | NHMe | H |
| I-600 | O | H | H | HOX3 | H | H | H | H | NHMe | H |
| I-601 | O | H | H | H | HOX3 | H | H | H | NHMe | H |
| I-602 | O | HOX3 | H | H | H | H | H | H | H | NHMe |

**Table 10**

| | | | | | | | | | | |
|---|---|---|---|---|---|---|---|---|---|---|
| I-603 | O | H | HOX3 | H | H | H | H | H | H | NHMe |
| I-604 | O | H | H | HOX3 | H | H | H | H | H | NHMe |
| I-605 | O | H | H | H | HOX3 | H | H | H | H | NHMe |
| I-606 | NH | H | H | HOX | H | H | H | H | H | H |
| I-607 | NH | H | H | HOX | H | H | H | NH₂ | H | H |
| I-608 | NH | H | H | HOX | H | H | H | OMe | H | H |
| I-609 | NH | H | H | HOX | H | H | H | OH | H | H |
| I-610 | NH | H | H | HOX2 | H | H | H | H | H | H |
| I-611 | NH | H | H | HOX2 | H | H | H | NH₂ | H | H |
| I-612 | NH | H | H | HOX2 | H | H | H | OMe | H | H |
| I-613 | NH | H | H | HOX2 | H | H | H | OH | H | H |
| I-614 | NH | H | H | HOX3 | H | H | H | H | H | H |
| I-615 | NH | H | H | HOX3 | H | H | H | NH₂ | H | H |
| I-616 | NH | H | H | HOX3 | H | H | H | OMe | H | H |
| I-617 | NH | H | H | HOX3 | H | H | H | OH | H | H |
| I-618 | NH | H | H | H | H | H | H | HOX | H | H |
| I-619 | NH | H | H | H | H | H | H | HOX2 | H | H |
| I-620 | NH | H | H | H | H | H | H | HOX3 | H | H |
| I-621 | NH | HOX | H | H | H | NMe₂ | H | H | H | H |
| I-622 | NH | H | HOX | H | H | NMe₂ | H | H | H | H |
| I-623 | NH | H | H | HOX | H | NMe₂ | H | H | H | H |
| I-624 | NH | H | H | H | HOX | NMe₂ | H | H | H | H |
| I-625 | NH | HOX | H | H | H | H | NMe₂ | H | H | H |
| I-626 | NH | H | HOX | H | H | H | NMe₂ | H | H | H |
| I-627 | NH | H | H | HOX | H | H | NMe₂ | H | H | H |
| I-628 | NH | H | H | H | HOX | H | NMe₂ | H | H | H |
| I-629 | NH | HOX | H | H | H | H | H | NMe₂ | H | H |
| I-630 | NH | H | HOX | H | H | H | H | NMe₂ | H | H |
| I-631 | NH | H | H | HOX | H | H | H | NMe₂ | H | H |
| I-632 | NH | H | H | H | HOX | H | H | NMe₂ | H | H |
| I-633 | NH | HOX | H | H | H | H | H | H | NMe₂ | H |
| I-634 | NH | H | HOX | H | H | H | H | H | NMe₂ | H |
| I-635 | NH | H | H | HOX | H | H | H | H | NMe₂ | H |
| I-636 | NH | H | H | H | HOX | H | H | H | NMe₂ | H |
| I-637 | NH | HOX | H | H | H | H | H | H | H | NMe₂ |
| I-638 | NH | H | HOX | H | H | H | H | H | H | NMe₂ |
| I-639 | NH | H | H | HOX | H | H | H | H | H | NMe₂ |
| I-640 | NH | H | H | H | HOX | H | H | H | H | NMe₂ |
| I-641 | NH | HOX | H | H | H | NHMe | H | H | H | H |
| I-642 | NH | H | HOX | H | H | NHMe | H | H | H | H |
| I-643 | NH | H | H | HOX | H | NHMe | H | H | H | H |
| I-644 | NH | H | H | H | HOX | NHMe | H | H | H | H |
| I-645 | NH | HOX | H | H | H | H | NHMe | H | H | H |
| I-646 | NH | H | HOX | H | H | H | NHMe | H | H | H |
| I-647 | NH | H | H | HOX | H | H | NHMe | H | H | H |
| I-648 | NH | H | H | H | HOX | H | NHMe | H | H | H |
| I-649 | NH | HOX | H | H | H | H | H | NHMe | H | H |
| I-650 | NH | H | HOX | H | H | H | H | NHMe | H | H |
| I-651 | NH | H | H | HOX | H | H | H | NHMe | H | H |
| I-652 | NH | H | H | H | HOX | H | H | NHMe | H | H |
| I-653 | NH | HOX | H | H | H | H | H | H | NHMe | H |
| I-654 | NH | H | HOX | H | H | H | H | H | NHMe | H |
| I-655 | NH | H | H | HOX | H | H | H | H | NHMe | H |
| I-656 | NH | H | H | H | HOX | H | H | H | NHMe | H |
| I-657 | NH | HOX | H | H | H | H | H | H | H | NHMe |
| I-658 | NH | H | HOX | H | H | H | H | H | H | NHMe |
| I-659 | NH | H | H | HOX | H | H | H | H | H | NHMe |
| I-660 | NH | H | H | H | HOX | H | H | H | H | NHMe |
| I-661 | NH | HOX2 | H | H | H | NMe₂ | H | H | H | H |
| I-662 | NH | H | HOX2 | H | H | NMe₂ | H | H | H | H |
| I-663 | NH | H | H | HOX2 | H | NMe₂ | H | H | H | H |
| I-664 | NH | H | H | H | HOX2 | NMe₂ | H | H | H | H |
| I-665 | NH | HOX2 | H | H | H | H | NMe₂ | H | H | H |
| I-666 | NH | H | HOX2 | H | H | H | NMe₂ | H | H | H |
| I-667 | NH | H | H | HOX2 | H | H | NMe₂ | H | H | H |
| I-668 | NH | H | H | H | HOX2 | H | NMe₂ | H | H | H |
| I-669 | NH | HOX2 | H | H | H | H | H | NMe₂ | H | H |

**Table 11**

| | | | | | | | | | | |
|---|---|---|---|---|---|---|---|---|---|---|
| I-670 | NH | H | HOX2 | H | H | H | H | NMe₂ | H | H |
| I-671 | NH | H | H | HOX2 | H | H | H | NMe₂ | H | H |
| I-672 | NH | H | H | H | HOX2 | H | H | NMe₂ | H | H |
| I-673 | NH | HOX2 | H | H | H | H | H | H | NMe₂ | H |
| I-674 | NH | H | HOX2 | H | H | H | H | H | NMe₂ | H |
| I-675 | NH | H | H | HOX2 | H | H | H | H | NMe₂ | H |
| I-676 | NH | H | H | H | HOX2 | H | H | H | NMe₂ | H |
| I-677 | NH | HOX2 | H | H | H | H | H | H | H | NMe₂ |
| I-678 | NH | H | HOX2 | H | H | H | H | H | H | NMe₂ |
| I-679 | NH | H | H | HOX2 | H | H | H | H | H | NMe₂ |
| I-680 | NH | H | H | H | HOX2 | H | H | H | H | NMe₂ |
| I-681 | NH | HOX2 | H | H | H | NHMe | H | H | H | H |
| I-682 | NH | H | HOX2 | H | H | NHMe | H | H | H | H |
| I-683 | NH | H | H | HOX2 | H | NHMe | H | H | H | H |
| I-684 | NH | H | H | H | HOX2 | NHMe | H | H | H | H |
| I-685 | NH | HOX2 | H | H | H | H | NHMe | H | H | H |
| I-686 | NH | H | HOX2 | H | H | H | NHMe | H | H | H |
| I-687 | NH | H | H | HOX2 | H | H | NHMe | H | H | H |
| I-688 | NH | H | H | H | HOX2 | H | NHMe | H | H | H |
| I-689 | NH | HOX2 | H | H | H | H | H | NHMe | H | H |
| I-690 | NH | H | HOX2 | H | H | H | H | NHMe | H | H |
| I-691 | NH | H | H | HOX2 | H | H | H | NHMe | H | H |
| I-692 | NH | H | H | H | HOX2 | H | H | NHMe | H | H |
| I-693 | NH | HOX2 | H | H | H | H | H | H | NHMe | H |
| I-694 | NH | H | HOX2 | H | H | H | H | H | NHMe | H |
| I-695 | NH | H | H | HOX2 | H | H | H | H | NHMe | H |
| I-696 | NH | H | H | H | HOX2 | H | H | H | NHMe | H |
| I-697 | NH | HOX2 | H | H | H | H | H | H | H | NHMe |
| I-698 | NH | H | HOX2 | H | H | H | H | H | H | NHMe |
| I-699 | NH | H | H | HOX2 | H | H | H | H | H | NHMe |
| I-700 | NH | H | H | H | HOX2 | H | H | H | H | NHMe |
| I-701 | NH | HOX3 | H | H | H | NMe₂ | H | H | H | H |
| I-702 | NH | H | HOX3 | H | H | NMe₂ | H | H | H | H |
| I-703 | NH | H | H | HOX3 | H | NMe₂ | H | H | H | H |
| I-704 | NH | H | H | H | HOX3 | NMe₂ | H | H | H | H |
| I-705 | NH | HOX3 | H | H | H | H | NMe₂ | H | H | H |
| I-706 | NH | H | HOX3 | H | H | H | NMe₂ | H | H | H |
| I-707 | NH | H | H | HOX3 | H | H | NMe₂ | H | H | H |
| I-708 | NH | H | H | H | HOX3 | H | NMe₂ | H | H | H |
| I-709 | NH | HOX3 | H | H | H | H | H | NMe₂ | H | H |
| I-710 | NH | H | HOX3 | H | H | H | H | NMe₂ | H | H |
| I-711 | NH | H | H | HOX3 | H | H | H | NMe₂ | H | H |
| I-712 | NH | H | H | H | HOX3 | H | H | NMe₂ | H | H |
| I-713 | NH | HOX3 | H | H | H | H | H | H | NMe₂ | H |
| I-714 | NH | H | HOX3 | H | H | H | H | H | NMe₂ | H |
| I-715 | NH | H | H | HOX3 | H | H | H | H | NMe₂ | H |
| I-716 | NH | H | H | H | HOX3 | H | H | H | NMe₂ | H |
| I-717 | NH | HOX3 | H | H | H | H | H | H | H | NMe₂ |
| I-718 | NH | H | HOX3 | H | H | H | H | H | H | NMe₂ |
| I-719 | NH | H | H | HOX3 | H | H | H | H | H | NMe₂ |
| I-720 | NH | H | H | H | HOX3 | H | H | H | H | NMe₂ |
| I-721 | NH | HOX3 | H | H | H | NHMe | H | H | H | H |
| I-722 | NH | H | HOX3 | H | H | NHMe | H | H | H | H |
| I-723 | NH | H | H | HOX3 | H | NHMe | H | H | H | H |
| I-724 | NH | H | H | H | HOX3 | NHMe | H | H | H | H |
| I-725 | NH | HOX3 | H | H | H | H | NHMe | H | H | H |
| I-726 | NH | H | HOX3 | H | H | H | NHMe | H | H | H |
| I-727 | NH | H | H | HOX3 | H | H | NHMe | H | H | H |
| I-728 | NH | H | H | H | HOX3 | H | NHMe | H | H | H |
| I-729 | NH | HOX3 | H | H | H | H | H | NHMe | H | H |
| I-730 | NH | H | HOX3 | H | H | H | H | NHMe | H | H |
| I-731 | NH | H | H | HOX3 | H | H | H | NHMe | H | H |
| I-732 | NH | H | H | H | HOX3 | H | H | NHMe | H | H |
| I-733 | NH | HOX3 | H | H | H | H | H | H | NHMe | H |
| I-734 | NH | H | HOX3 | H | H | H | H | H | NHMe | H |
| I-735 | NH | H | H | HOX3 | H | H | H | H | NHMe | H |
| I-736 | NH | H | H | H | HOX3 | H | H | H | NHMe | H |

**Table 12**

| | | | | | | | | | | |
|---|---|---|---|---|---|---|---|---|---|---|
| I-737 | NH | HOX3 | H | H | H | H | H | H | H | NHMe |
| I-738 | NH | H | HOX3 | H | H | H | H | H | H | NHMe |
| I-739 | NH | H | H | HOX3 | H | H | H | H | H | NHMe |
| I-740 | NH | H | H | H | HOX3 | H | H | H | H | NHMe |
| I-741 | O | I | H | H | H | H | H | HOX | H | H |
| I-742 | O | I | H | H | H | H | H | HOX2 | H | H |
| I-743 | O | I | H | H | H | H | H | HOX3 | H | H |
| I-744 | O | I | H | H | H | H | H | FX | H | H |
| I-745 | O | I | H | H | H | H | H | FX2 | H | H |
| I-746 | O | I | H | H | H | H | H | FX3 | H | H |
| I-747 | NH | I | H | H | H | H | H | HOX | H | H |
| I-748 | NH | I | H | H | H | H | H | HOX2 | H | H |
| I-749 | NH | I | H | H | H | H | H | HOX3 | H | H |
| I-750 | NH | I | H | H | H | H | H | FX | H | H |
| I-751 | NH | I | H | H | H | H | H | FX2 | H | H |
| I-752 | NH | I | H | H | H | H | H | FX3 | H | H |

Among the above-described compounds, I-6 (compound 14), I-7 (compound 17), I-8 (compound 20), I-86 (compound 7), I-106 (compound 12), I-741 (compound 26), I-742 (compound 27) and I-743 (compound 28) are preferred. Among them, I-86 and I-106 are more preferable.

The compound represented by general formula (I) may be synthesized according to the descriptions in the Examples provided later, Varma RS et al, Tetrahedron Letters, 33(40), 5937-40, 1992, and the like.

The compound represented by general formula (I) is preferably labeled with a labeling substance. As the labeling substance, fluorescent substances, affinity-substances, and the like may be used. Preferably, radionuclides are used. Types of radionuclides used for labeling are not particularly limited, and may be suitably selected depending on the embodiment of use. For example, when the compound represented by general formula (I) is used for diagnosis by single photon emission computed tomography (SPECT), γ-ray-emitting radionuclides such as ^{99m}Tc, ¹¹¹In, ⁶⁷Ga, ²⁰¹TI, ¹²³I, and ¹³³Xe (preferably, ^{99m}Tc and ¹²³I) may be used as the radionuclides. Furthermore, when the compound represented by the general formula (I) is used for diagnosis by positron emission tomography (PET), positron-emitting radionuclides such as ¹¹C, ¹³N, ¹⁵O, ¹⁸F, ⁶²Cu, ⁶⁸Ga, and ⁷⁶Br (preferably, ¹¹C, ¹³N, ¹⁵O, and ¹⁸F) may be used. Furthermore, when the compound represented by general formula (I) is administered to animals other than human, radionuclides having a longer half life, such as ¹²⁵I, may be used. Radionuclides may be contained in the molecule of the compound represented by general formula (I) or bound to the compound represented by general formula (I).

As the method for binding a radionuclide to the compound represented by general formula (I), a method commonly used for each radionuclide may be used. Furthermore, when a radionuclide is bound to the compound represented by general formula (I), the radionuclide alone may be bound. Alternatively, a radionuclide bound to another substance may be bound. Since the above-mentioned ^{99m}Tc is usually bound to a compound to be labeled in the form of a complex, a complex containing ^{99m}Tc may be bound to the compound represented by general formula (I). Examples of complexes containing ^{99m}Tc include a complex containing 2-hydrazinopyridine (Liu S et al., Bioconjug Chem. 1996 Jan-Feb; 7(1): 63-71), a complex containing N-(2-mercaptoethyl)-2-[(2-mercaptoethyl)amino]-acetamide (Zhen W et al., J Med Chem. 1999 Jul 29; 42(15): 2805-15), a complex containing 2,2'-(1,2-ethanediyldiimino)bis-ethanethiol (Oya S et al., Nucl Med Biol. 1998 Feb; 25(2): 135-40), a tricarbonyl complex (Schibli R et al., Bioconjug Chem. 2000 may-Jun; 11(3): 345-51), and so forth.

Instead of the compound represented by general formula (I), a pharmaceutically acceptable salt thereof may be used. Examples of pharmaceutically acceptable salts include alkali metal salts (sodium salts, potassium salts, and lithium salts), alkaline earth metal salts (calcium salts and magnesium salts), sulfates, hydrochlorides, nitrates, phosphates, and so forth.

The composition of the present invention is used for diagnosing an amyloid-related disease. Here, the term "amyloid-related disease" means a disease caused by accumulation of amyloid β protein, primarily Alzheimer's disease; this term also includes diseases such as Down's syndrome, hereditary cerebral hemorrhage with amyloidosis-Dutch type (HCHWA-D), etc. Furthermore, prodromes of such diseases, which are not generally recognized as a "disease," are also included in the "amyloid-related disease" in the present invention. As an examples of such a prodrome, mild cognitive impairment (MCI) observed before the onset of Alzheimer's disease may be given.

Usually, diagnosis of an amyloid-related disease using the composition of the present invention is made by administering the composition of the present invention to a patient for whom a diagnosis is to be made, a laboratory animal, or the like, then imaging the brain thereof, and making diagnosis based on the state of the compound represented by general formula (I) (quantity, distribution, etc.) in the image. The administration method of the composition of the present invention is not particularly limited and may be suitably selected depending on the type of a compound, the type of a labeling substance, and the like. Usually, the composition of the present invention is administered by injection, drip infusion, or the like into the skin, the peritoneal cavity, the vein, the artery, or the spinal fluid. The dose of the composition of the present invention is not particularly limited and may be suitably selected depending on the type of a compound, the type of a labeling substance, and the like. For adults, preferably 10⁻¹⁰ to 10⁻³ mg, more preferably 10⁻⁸ to 10⁻⁵ mg of the compound represented by general formula (I) per day is administered.

Since the composition of the present invention is usually administered by injection or drip infusion as described above, components usually contained in injection solutions or drip infusion solutions may be contained in the composition. Examples of such components include liquid carriers (for example, potassium phosphate buffer, physiological saline, Ringer's solution, distilled water, polyethylene glycol, vegetable oils and fats, ethanol, glycerine, dimethyl sulfoxide, propylene glycol, etc.), antibacterial agents, local anesthetics (for example, procaine hydrochloride, dibucaine hydrochloride, etc.), buffers (for example, Tris-hydrochloride buffer, HEPES buffer, etc.), and osmotic modifiers (for example, glucose, sorbitol, sodium chloride, etc.).

The composition for diagnosing amyloid-related diseases of the present invention may also be used for screening for therapeutic or prophylactic agents for amyloid-related diseases. For example, a test substance is administered to a model animal of a "disease" such as Alzheimer's disease; the composition for diagnosing amyloid-related diseases of the present invention is administered to the model animal; and then the distribution or the quantity of the compound represented by general formula (I) contained in the brain of the model animal is examined. As a result, when a significant difference (for example, a reduced distribution site, a decreased quantity, etc.) is detected as compared with a control (model animal not receiving the test substance), the test substance can be a candidate therapeutic agent for the amyloid-related disease. Furthermore, after a test substance is administered to a model animal of a "prodrome of a disease" such as mild cognitive impairment, the composition for diagnosing amyloid-related diseases of the present invention is administered to the model animal, and then the distribution or the quantity of the compound represented by general formula (I) contained in the brain of the model animal is examined. As a result, when a significant difference (for example, a reduction or a slowed enlargement of distribution site, a decrease or a slowed increase in quantity, etc.) is detected as compared with a control, the test substance can be a candidate prophylactic agent for the amyloid-related disease.

Furthermore, the composition for diagnosing amyloid-related diseases of the present invention may also be used to evaluate therapeutic or prophylactic agents for amyloid-related diseases whose effects have already been confirmed. Specifically, after the therapeutic or prophylactic agent for the disease is administered to a model animal of an amyloid-related disease, the composition for diagnosing amyloid-related diseases of the present invention is administered to the model animal, and then the distribution or the quantity of the compound represented by general formula (I) contained in the brain of the model animal is examined. This allows evaluation of the above-mentioned therapeutic or prophylactic agent (specifically, effective dosage, effective administration method, etc.).

### EXAMPLES

Hereafter, the present invention will be explained more specifically with reference to the following examples.

### EXAMPLE 1

### Experimental Methods

### (1) Reagents and Instruments

As radioactive iodine-125 (¹²⁵I), IODINE-125 (185 MBq) from Amersham Bioscience was used. For reversed-phase HPLC, Cosmosil 5C18-AR column (4.6 x 150 mm) from Nacalai Tesque was used with an elution solvent of ultrapure water (A) and acetonitrile (B) mixed at A:B=30:70, at a flow rate of 1.0 mL/min. Mass spectra were obtained on a JEOL IMS-DX instrument. Amyloid β protein (Human, 1-42) [TFA form] was purchased from Peptide Institute, Inc. Other reagents were of special grade. ¹H-NMR spectra were obtained on a Varian Gemini 300 spectrometer with tetramethylsilane as an internal standard.

### (2) Synthesis of Aurone Derivatives

### Synthesis of 2-(2-(methoxy-2-oxyethoxy)-5-bromobenzoic acid methyl ester (Compound 1)

To a solution of 2-hydroxy-5-bromobenzoic acid methyl ester (1.5 g, 6.49 mmol) in acetone (10 mL) was added K₂CO₃ (2.7 g). Subsequently, ethylbromoacetate (1.3 mL) was added thereto dropwise under stirring, and the mixture was heated to reflux for 3 h. After completion of the reaction was completed, the solvent was evaporated. The residue was dissolved in purified water (100 mL) and extracted with ethyl acetate (100 mL). After the extract was dried over anhydrous sodium sulfate, evaporation of the solvent gave Compound 1. Yield: 1.60 g (yield rate: 77.7%). ¹H NMR NMR (300 MHz, CDCl₃) d 1.29 (t, J = 7.2 Hz, 3H), 3.91 (s, 3H), 4.24 (q, J = 7.2 Hz, 2H), 4.69 (s, 2H), 6.78 (d, J = 9.0 Hz, 1H), 7.54 (d, J = 6.3 Hz, 1H), 7.96 (s, 1H).

### Synthesis of 2-(carboxymethoxy)-5-bromobenzoic acid (Compound 2)

To a solution of Compound 1 (1.6 g, 5.04 mmol) in methanol (10 mL) was added 10% NaOH (3.0 mL). The mixture was reacted for 2 h. After completion of the reaction, 1 N HCl was added under cooling. The resultant crystals were filtered to give Compound 2. Yield: 1.10 g (yield rate: 79.4%).

### Synthesis of 5-bromo-3-acetoxybenzofuran (Compound 3)

In a mixture of acetic anhydride (20 mL) and acetic acid (4 mL), Compound 2 (1.10 g, 4.00 mmol) was dissolved. After addition of sodium acetate (1.0 g) thereto, the mixture was heated to reflux for 5 h. After completion of the reaction, purified water (100 mL) was added, and the reaction mixture was extracted with chloroform (100 mL) to give Compound 3. Yield: 0.70 g (yield rate: 68.4%) ¹H NMR (300 MHz, CDCl₃) d 2.37 (s, 3H), 7.33 (d, J = 9.0 Hz, 1H), 7.43 (dd, J = 3.6, 2.1 Hz, 1H), 4.69 (s, 2H), 7.71 (s, 1H), 8.02 (s, 1H).

### Synthesis of 5-bromo-3(2H)-benzofuranone (Compound 4)

A mixture of Compound 3, methanol (10 mL), purified water (5 mL), and 1 N HCl (2 mL) was heated to reflux for 3 h under stirring. After completion of the reaction, the reaction solution was cooled and purified water (100 ml) was added thereto. The crystals formed were collected by vacuum filtration to give Compound 4. Yield: 0.50 g (yield rate: 86.9 %) ¹H NMR (300 MHz, CDCl₃) d 4.67 (s, 2H), 7.06 (d, J = 9.0 Hz, 1H), 7.69 (dd, J = 2.1, 2.1 Hz, 1H), 7.79 (d, J = 2.1 Hz, 1H).

### Synthesis of 5-bromo-4'-dimethylaminoaurone (Compound 5)

To a solution of Compound 4 (50 mg, 0.23 mmol) and 4-dimethylaminobenzaldehyde (35 mg, 0.235 mmol) in chloroform (5 mL) was added aluminum oxide (1.6 g). The mixture was stirred for 20 min. After completion of the reaction, the reaction mixture was extracted with chloroform (100 mL). Evaporation of the solvent gave Compound 5. Yield: 42 mg (yield rate: 51.9 %) ¹H NMR (300 MHz, CDCl₃) d 3.09 (s, 6H), 6.70 (d, J = 11.4 Hz, 2H), 6.95 (s, 1H), 7.22 (d, J = 8.7 Hz, 1H), 7.67 (d, J =9.6Hz, 1H), 7.83 (d, J =9.0 Hz, 2H), 7.92 (d, J =2.1 Hz, 1H).

### Synthesis of 5-tributyltin-4'-dimethylaminoaurone (Compound 6)

To a solution of Compound 5 (12 mg, 0.035 mmol) in dioxane (5 mL), bis(tributyltin) (0.3 mL), tetra-triphenylphosphinepalladium (50 mg, 0.043 mmol) and triethylamine (5 mL) were added. The mixture was heated at 90°C for 3 h. After the solvent was evaporated, the residue was subjected to silica gel column chromatography with an elution solvent of ethyl acetate/hexane (1/4) to thereby obtain Compound 6. Yield: 7.0 mg (yield rate: 35.2%) ¹H NMR (300 MHz, CDCl₃) d 0.86-1.57 (m, 27H), 3.07 (s, 6H), 6.75 (d, J = 9.0 Hz, 2H), 6.92 (s, 1H), 7.29 (d, J = 7.2 Hz, 1H), 7.67 (dd, J =1.2, 0.9 Hz, 1H), 7.86 (d, J =9.0 Hz, 2H), 7.95 (s, 1H).

### Synthesis of 5-iodo-4'-dimethylaminoaurone (Compound 7)

To a solution of Compound 6 (7 mg, 0.35 mmol) in chloroform (3 mL), iodine in chloroform (0.7 mL, 0.25 M) was added at room temperature under stirring. After 10 min reaction at room temperature, saturated aqueous solution of sodium hydrogen sulfite (15 mL) was added to terminate the reaction. The chloroform layer was separated and dried with sodium sulfate. After evaporation of the solvent, the residue was subjected to silica gel chromatography using ethyl acetate/hexane (1/4) as an elution solvent to thereby obtain 7. Yield: 3.0 mg (yield rate: 63.9%) ¹H NMR (300 MHz, CDCl₃) d 3.08 (s, 6H), 6.74 (d, J = 9.0 Hz, 2H), 6.95 (s, 1H), 7.12 (d, J = 8.7 Hz, 1H), 7.82-7.88 (m, 3H), 8.12 (s, 1H). MS m/z 391 (M⁺).

### Synthesis of 5-bromo-4'-nitroaurone (Compound 8)

To a solution of Compound 4 (100 mg, 0.47 mmol) and 4-nitrobenzaldehyde (80 mg, 0.53 mmol) in chloroform, aluminum oxide (3.0 g) was added and the mixture was stirred for 20 min. After completion of the reaction, the reaction mixture was extracted with chloroform. Then, evaporation of the solvent gave Compound 8. Yield: 100 mg (yield rate: 61.6%).

### Synthesis of 5-bromo-4'-aminoaurone (Compound 9)

To a solution of Compound 8 (10 mg, 0.028 mmol) in ethanol (10 mL), tin(II) chloride (0.64 g, 2.37 mmol) was added gradually under stirring. The mixture was heated to reflux for 2 h. After completion of the reaction, 1 N aqueous sodium hydroxide (100 mL) was added. Then, the reaction mixture was extracted with ethyl acetate (100 mL) and dried over anhydrous sodium acetate. Evaporation of the solvent gave Compound 9. Yield: 7.0 mg (yield rate: 79.1%) ¹H NMR (300 MHz, CDCl₃) d 4.11 (s, 2H), 6.72 (d, J = 8.7 Hz, 2H), 6.90 (s, 1H), 7.23 (d, J = 8.4 Hz, 1H), 7.70 (dd, J =2.1, 2.1 Hz, 1H), 7.76 (d, J =8.4 Hz, 2H), 7.92 (d, J =1.8 Hz, 1H).

### Synthesis of 5-bromo-4'-methylaminoaurone (Compound 10)

Compound 9 (100 mg, 0.316 mmol) was dissolved in DMSO (7 mL). Subsequently, K₂CO₃ (380 mg) and CH₃I (0.15 mL) were added thereto and the mixture was stirred at 50°C for 3 h. After completion of the reaction, purified water (100 mL) was added. The reaction mixture was extracted with ethyl acetate (100 mL). The solvent was evaporated and the residue was subjected to silica gel column chromatography using ethyl acetate/hexane (1/2) as an elution solvent, to thereby obtain Compound 10. Yield: 49 mg (yield rate: 47.5%) ¹H NMR (300 MHz, CDCl₃) d 2.92 (s, 3H), 4.21 (s, 1H), 6.59 (d, J = 8.7 Hz, 2H), 6.92 (s, 1H), 7.25 (d, J = 8.7 Hz, 1H), 7.68 (dd, J =2.1, 2.1 Hz, 1H), 7.78 (d, J =8.7 Hz, 2H), 7.91 (d, J =2.1 Hz, 1H).

### Synthesis of 5-tributyltin-4'-methylaminoaurone (Compound 11)

To a solution of Compound 10 (49 mg, 0.15 mmol) in dioxane (5 mL), bis(tributyltin) (0.4 mL), tetra-triphenylphosphinepalladium (50 mg, 0.043 mmol) and triethylamine (5 mL) were added. The mixture was heated at 90°C for 5 h. After evaporation of the solvent, the residue was subjected to silica gel column chromatography using ethyl acetate/hexane (1/2) as an elution solvent, to thereby obtain Compound 11. Yield: 28 mg (yield rate: 34.6 %) ¹H NMR (300 MHz, CDCl₃) d 1.06-1.57 (m, 27H), 3.12. (s, 3H), 4.42 (s, 1H), 6.73 (d, J = 8.7 Hz, 2H), 7.10 (s, 1H), 7.49 (d, J = 8.1 Hz, 1H), 7.88 (dd, J =1.2, 1.2 Hz, 1H), 8.01 (d, J =8.7 Hz, 2H), 8.09 (s, 1H).

### Synthesis of 5-iodo-4'-methylaminoaurone (Compound 12)

To a solution of Compound 11 (30 mg, 0.055 mmol) in chloroform (3 mL), iodine in chloroform (1 mL, 0.25 M) was added at room temperature under stirring. After 10 min reaction at room temperature, saturated aqueous solution of sodium hydrogen sulfite (15 mL) was added to terminate the reaction. The chloroform layer was separated and dried with sodium sulfate. After evaporation of the solvent, the residue was subjected to silica gel column chromatography using ethyl acetate/hexane (1/2) as an elution solvent to thereby obtain 12. Yield: 10 mg (yield rate: 48.2 %) ¹H NMR (300 MHz, CDCl₃) d 2.92 (s, 3H), 4.27 (s, 1H), 6.64 (d, J = 9.0 Hz, 2H), 6.90 (s, 1H), 7.12 (d, J = 8.4 Hz, 1H), 7.79 (d, J =8.7 Hz, 2H), 7.86 (dd, J =2.1, 2.1 Hz, 1H), 8.11 (d, J =2.1 Hz, 1H).

### Synthesis of 5-tributyltin-4'-aminoaurone (Compound 13)

To a solution of Compound 9 (78 mg, 0.25 mmol) in dioxane (7 mL), bis(tributyltin) (0.4 mL, tetra-triphenylphosphinepalladium (50 mg, 0.043 mmol) and triethylamine (5 mL) were added. The mixture was heated at 90°C for 4 h. After evaporation of the solvent, the residue was subjected to silica gel column chromatography using ethyl acetate/hexane (1/2) as an elution solvent to thereby obtain 13. Yield: 30 mg (yield rate: 23.1 %) ¹H NMR (300 MHz, CDCl₃) d 0.86-1.57 (m, 27H), 4.05 (s, 2H), 6.73 (d, J = 8.7 Hz, 2H), 6.87 (s, 1H), 7.30 (d, J = 8.1 Hz, 1H), 7.69 (dd, J =0.6, 0.9 Hz, 1H), 7.78 (d, J =8.4 Hz, 2H), 7.89 (s, 1H).

### Synthesis of 5-iodo-4'-aminoauron (Compound 14)

To a solution of Compound 13 (30 mg, 0.057 mmol) in chloroform (3 mL), iodine in chloroform (1 mL, 0.25 M) was added at room temperature. After 10 min reaction at room temperature, saturated aqueous solution of sodium hydrogen sulfite (15 mL) was added to terminate the reaction. The chloroform layer was separated and dried with sodium sulfate. After evaporation of the solvent, the residue was subjected to silica gel column chromatography using ethyl acetate/hexane (1/2) as an elution solvent to thereby obtain 14. Yield: 14 mg (yield rate: 67.6 %) ¹H NMR (300 MHz, CDCl₃) d 4.10 (s, 2H), 6.72 (d, J = 8.7 Hz, 2H), 6.90 (s, 1H), 7.13 (d, J = 9.0 Hz, 1H), 7.76 (d, J =8.4 Hz, 2H), 7.87 (d, J =10.5 Hz, 1H), 8.11 (s, 1H).

### Synthesis of 5-bromo-4'-methoxyaurone (Compound 15)

To a solution of Compound 4 (300 mg, 1.41 mmol) and 4-methoxybenzaldehyde (192 mg, 1.41 mmol) in chloroform, aluminum oxide (5.0 g) was added, and the mixture was stirred for 20 min. After completion of the reaction, the reaction mixture was extracted with chloroform (100 mL). Evaporation of the solvent gave Compound 15. Yield: 410 mg (yield rate: 85.7 %) ¹H NMR (300 MHz, CDCl₃) d 3.88 (s, 3H), 6.91 (s, 1H), 6.99 (d, J = 9.0 Hz, 2H), 7.24 (d, J = 8.4 Hz, 1H), 7.73 (dd, J =2.1, 2.1 Hz, 1H), 7.88 (d, J =8.7 Hz, 2H), 7.92 (d, J =1.8 Hz, 1H).

### Synthesis of 5-tributyltin-4'-methoxyaurone (Compound 16)

To a solution of Compound 9 (200 mg, 0.60 mmol) in dioxane (5 mL), bis(tributyltin) (0.4 mL), tetra-triphenylphosphinepalladium (50 mg, 0.043 mmol) and triethylamine (5 mL) were added. The mixture was heated at 90°C for 24 h. After evaporation of the solvent, the residue was subjected to silica gel column chromatography using chloroform as an elution solvent to thereby obtain 16. Yield: 50 mg (yield rate: 15.3 %) ¹H NMR (300 MHz, CDCl₃) d 0.89-1.64 (m, 27H), 3.87 (s, 3H), 6.89 (s, 1H), 6.99 (d, J = 8.7 Hz, 2H), 7.31 (d, J = 8.1 Hz, 1H), 7.71 (dd, J =0.9, 0.9 Hz, 1H), 7.86 (d, J =9.0 Hz, 2H), 7.90 (d, J =8.7 Hz 1H).

### Synthesis of 5-iodo-4'-methoxyaurone (Compound 17)

To a solution of Compound 16 (10 mg, 0.35 mmol) in chloroform (3 mL), iodine in chloroform (1 mL, 0.25 M) was added at room temperature. After 10 min reaction at room temperature, saturated aqueous solution of sodium hydrogen sulfite (15 mL) was added to terminate the reaction. The chloroform layer was separated and dried with sodium sulfate. After evaporation of the solvent, the residue was subjected to silica gel column chromatography using chloroform as an elution solvent to thereby obtain 17. Yield: 7 mg (yield rate: 71.5 %) ¹H NMR (300 MHz, CDCl₃) d 3.88 (s, 3H), 6.91 (s, 1H), 6.98 (d, J = 6.9 Hz, 2H), 7.14 (d, J = 8.4 Hz, 1H), 7.87-7.91 (m, 3H), 8.12 (d, J =2.1 Hz, 1H).

### Synthesis of 5-bromo-4'-hydroxyaurone (Compound 18)

Compound 15 (300 mg, 0.91 mmol) was dissolved in dichloromethane (25 mL). Under ice-cooling, boron tribromide in dichloromethane (3.0 mL) was gradually added thereto. After reaction on ice, the reaction mixture was gradually added to ice water and extracted separately in dichloromethane layer and aqueous layer. After evaporation of the solvent, the residue was subjected to silica gel column chromatography using ethyl acetate/hexane (3/2) as an elution solvent to thereby obtain 18. Yield: 15 mg (yield rate: 5.2%).

### Synthesis of 5-tributyltin-4'-hydroxyaurone (Compound 19)

To a solution of Compound 18 (50 mg, 0.16 mmol) in dioxane (5 mL), bis(tributyltin) (0.4 mL), tetra-triphenylphosphinepalladium (50 mg, 0.043 mmol) and triethylamine (5 mL) were added. The mixture was heated at 90°C for 10 h. After evaporation of the solvent, the residue was subjected to silica gel column chromatography using ethyl acetate/hexane (3/2) as an elution solvent to thereby obtain 19. Yield: 10 mg (yield rate: 12.0 %) ¹H NMR (300 MHz, CDCl₃) d 0.86-1.60 (m, 27H), 6.74 (d, J = 9.0 Hz, 2H), 6.90 (s, 1H), 7.30 (d, J = 8.1 Hz, 1H), 7.69 (d, J =8.1 Hz, 1H), 7.80 (d, J =8.4 Hz, 2H), 7.89 (s, 1H).

### Synthesis of 5-iodo-4'-hydroxyaurone (Compound 20)

To a solution of Compound 19 (10 mg, 0.027 mmol) in chloroform (3 mL), iodine in chloroform(1 mL, 0.25 M) was added at room temperature. After 10 min reaction at room temperature, saturated aqueous solution of sodium hydrogen sulfite (15 mL) was added to terminate the reaction. The chloroform layer was separated and dried with sodium sulfate. After evaporation of the solvent, the residue was subjected to silica gel column chromatography using ethyl acetate/hexane (3/2) as an elution solvent to thereby obtain Compound 20. Yield: 6 mg (yield rate: 84.0 %).

### (3) In vitro Binding Experiment using Aβ (1-42) Aggregates

Aβ aggregates were prepared by dissolving in a buffer containing 10 mM sodium phosphate and 1 mM EDTA (pH 7.4) at a concentration of 0.5 mg/mL and incubating at 37°C for 42 h. The binding experiment was performed using 12 x 75 mm borosilicate glass tubes. [¹²⁵I]IMPY used for comparison was prepared according to the method described previously (Zhuang ZP, Kung MP, Wilson A, Lee CW, Plossl K, Hou C, Holzman DM, Kung HF. Structure-activity relationship of imidazo[1,2-a]pyridines as ligands for detecting β-amyloid plaques in the brain. J. Med. Chem. 2003, 46(2): 237-43). 900 µL of 10% ethanol solution, 50 µL of [¹²⁵I]-labeled aurone derivative with various concentrations (in 10% aqueous ethanol) or 50 µL of [¹²⁵I]IMPY, and 50 µL (29 nM) of an Aβ(1-42) aggregate solution were mixed, and the mixture was allowed to stand at room temperature for 3 h. Aβ aggregate-bound aurone derivative and nonbound aurone derivative were separated with a Brandel M-24R cell harvester using Whatman GF/B filters. The radioactivities of substances remaining on the filter used for filtration were measured with a γ counter.

### (4) In vivo Radioactivity Biodistribution Experiment in Normal Mice

¹²⁵I-Iabeled compounds were diluted with 10% ethanol-containing physiological saline. Five 5-week-old male ddY mice per group were decapitated at 2, 10, 30, or 60 min after intravenous administration of 100 µL (0.5 to 1 µCi) of the labeled compound. After their blood was collected, organs were removed, and the weights and radioactivities thereof were measured.

### (5) Autoradiography Using Brain Tissue Sections from Alzheimer's Disease Patients

Brain tissue sections from Alzheimer's disease patients (hippocampus, paraffin sections, 5 µm) were purchased from BioChain. These sections were dewaxed by washing in xylene (5 min, twice) and in 100% ethanol, 100% ethanol, 95% ethanol, 85% ethanol and 70% ethanol (each 1 min, once). Autoradiography was carried out by reacting the section with ¹²⁵I-Iabeled aurone compound (5-iodo-4'-aminoaurone; Compound 14) (0.2 nM) prepared in advance. After the reaction, each section was washed in a solution of saturated lithium carbonate in 40% aqueous ethanol (2 min, twice), in 40% ethanol (2 min, once) and in water (30 sec, once). After air drying, the section was fixed on an imaging plate (Fuji Film) for 48 h, followed by analysis with BAS500 (Fuji Film). Immunostaining of senile plaque amyloid was performed using sections adjacent to the brain section used in autoradiography and according to the technique described in Amyloid β Immunostaining Kit (Wako Purechemical).

### [Experimental Results]

### (1) Synthesis of Aurone Derivatives

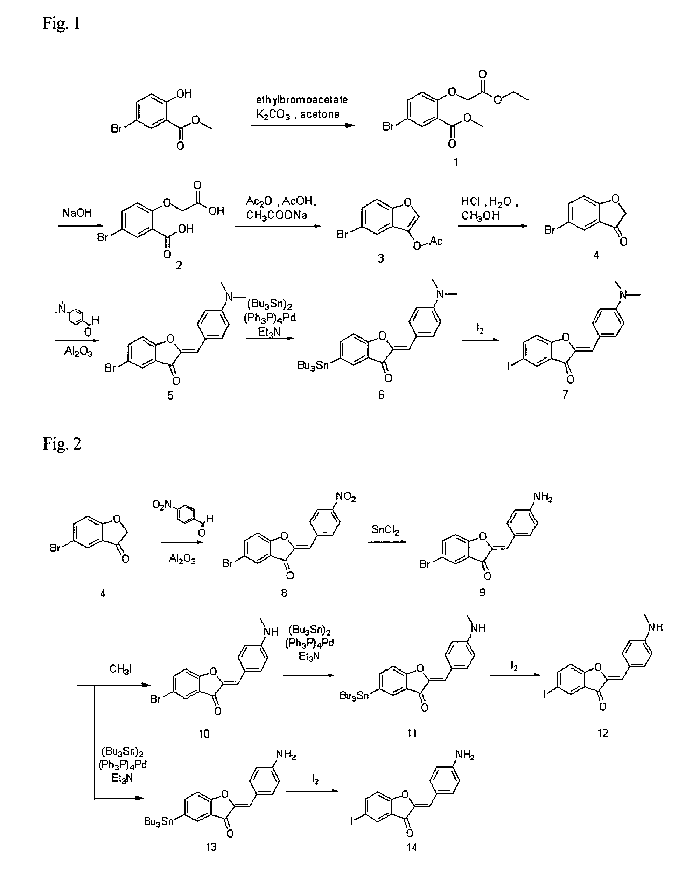

Figs. 1, 2 and 3 show synthetic pathways for aurone derivatives. The aurone skeleton was formed by aldol condensation reaction of benzofuranone (synthesized by a conventional method) and aldehyde. Compounds 5, 9, 10, 15 and 18 were converted to tributyltin forms through a reaction with bis(tributyltin) using palladium as a catalyst. Compounds 6, 11, 13, 16 and 19 in tributyltin forms were converted to Compounds 7, 12, 14, 17 and 20 through a reaction with iodine. Further, the tributyltin compounds were labeled with ¹²⁵I through a conventional tributyltin-iodine exchange reaction. Briefly, 50 µL of a tributyltin compound (1.0 mg/ml ethanol solution) was placed in a glass vial to which 1-2 µL of [¹²⁵I] NaI (3700-7400 kBq), 50 µL of 1 N HCl and 50 µL of 3% hydrogen peroxide were added. The resultant mixture was left at room temperature for 1 to 3 min. Then, 100 µL of saturated aqueous sodium hydrogen sulfite and 100 µL of saturated aqueous sodium hydrogen carbonate were added thereto to terminate the reaction. After addition of ethyl acetate (1 mL), the reaction mixture was fed to a Pasteur pipette packed with sodium sulfate for dehydration. The ethyl acetate was evaporated under nitrogen gas flow. The residue was dissolved in 100 µl of ethanol and purified by reversed-phase HPLC using water:acetonitrile (3:7) as an elution solvent, to thereby obtain a ¹²⁵I-Iabelled aurone derivative of interest.

### (2) Binding Experiment ofAurone Derivatives to Ab (1-42) Aggregates

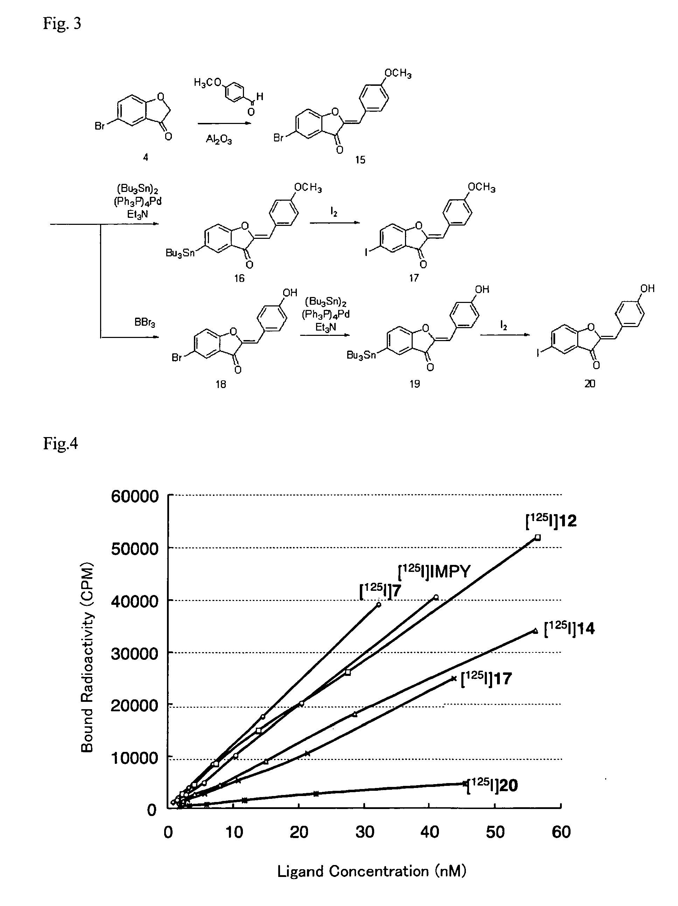

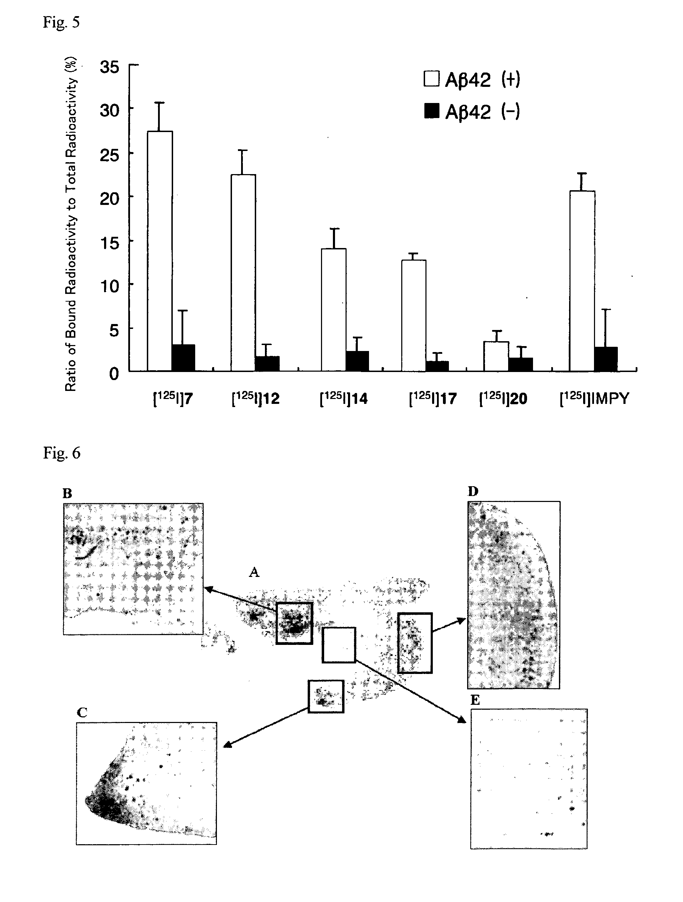

Figs. 4 and 5 show the results of experiments in which aurone derivatives were reacted in the presence and absence of Ab aggregates; the reaction mixture was filtered with a cell harvester; and the remaining radioactivity on the filter was measured. While the remaining radioactivity on the filter was low in any aurone derivatives in the absence of Ab aggregates, ¹²⁵I-Iabeled aurone derivatives showed remarkably high radioactivities in the presence of Ab aggregates (Fig. 5). These results revealed that any aurone derivative possesses a high binding property to Ab aggregates, though the property varies depending of the type of the substituent introduced into their side chains. The intensity of the binding property increased in the following order: [¹²⁵I] Compound 7 > [¹²⁵I] Compound 12 = [¹²⁵I] IMPY > [¹²⁵I] Compound 14 = [¹²⁵I] Compound 17 > [¹²⁵I] Compound 20 (Fig. 4). The binding property to Ab aggregates of those compounds with -NH₂, OMe- and OH group in their side chains are lower than the binding property of [¹²⁵I] IMPY. On the other hand, those compounds with NHMe- and NMe₂ group show equal or higher binding property to Ab aggregates compared to the binding property of [¹²⁵I] IMPY. All the 5 types of aurone derivatives tested this time showed a very high binding property to Ab aggregates. Among all, the binding of Compound 7 to Ab aggregates was equal or more than the binding of [¹²⁵I] IMPY which is clinically tested at present. Therefore, it has become clear that this compound satisfies a critical requirement as an imaging probe for amyloid β.

### (3) In vivo Radioactivity Distribution Experiment in Mice

Fig. 13 shows biodistribution of radioactivity in normal mice after the injection of aurone derivatives. When any of the aurone derivatives was used, sufficient radioactivity to perform imaging of intracerebral amyloid migrated to the brain from the early stage of injection (1.7-4.6% ID/g). Subsequently, rapid clearance of radioactivity was observed with the passage of time. The radioactivity remaining in the brain at 30 min after injection was 7-12% of the radioactivity remaining at 2 min after injection; and the radioactivity remaining in the brain at 60 min after injection was 2-8% of the radioactivity remaining at 2 min after injection. These results suggested that when aurone derivatives are applied to Alzheimer's disease model mice or Alzheimer's disease patients, they should exhibit a high binding property to amyloid deposited sites and rapid clearance of radioactivity from normal sites in the brain suffering from Alzheimer's disease; thus, aurone derivatives are capable of achieving a high S/N ratio. It was also shown that, in any of the aurone derivatives, *in vivo* radioactivity behavior is discharged mainly from the liver to the bile duct and the intestinal tract.

**Table 13 Biodistribution of Radioactivity in Normal Mice^{a}**

| | Time (min) after Injection | | | |
|---|---|---|---|---|
| Tissue | 2 | 10 | 30 | 60 |
| | [¹²⁵I]7 | | | |
| Blood | 2.69 (0.29) | 196 (0.34) | 1.48 (0.15) | 0.94 (0.25) |
| Liver | 10.21 (1.87) | 7.86 (2.25) | 5.60 (0.59) | 4.33 (0.76) |
| Kidney | 7.84 (1.92) | 8.31 (3.19) | 10.37 (2.34) | 4.51 (1.43) |
| Intestine | 1.65 (0.35) | 5.37 (1.10) | 9.50 (1.45) | 11.85 (2.70) |
| Spleen | 1.76 (0.28) | 0.81 (0.54) | 0.51 (0.10) | 0.37 (0.11) |
| Lung | 4.02 0.83) | 2.44 (0.54) | 1.51 (0.41) | 1.17 (0.26) |
| Stomach*^{b}* | 1.21 (0.29) | 1.35 (1.25) | 1.87 (0.79) | 1.51 (1.04) |
| Pancreas | 3.17 (0.68) | 1.04 (0.25) | 0.55 (0.21) | 0.30 (0.06) |
| Heart | 3.80 (0.66) | 1.36 (0.66) | 0.55 (0.29) | 0.45 (0.11) |
| Brain | 1.89 (0.38) | 0.69 (0.21) | 0.26 (0.04) | 0.11 (0.03) |
| | [¹²⁵I]12 | | | |
| Blood | 4.16 (0.98) | 2.72 (1.01) | 2.84 (1.48) | 2.41 (0.70) |
| Liver | 11.26 (2.81) | 9.70 (2.78) | 6.11 (2.16) | 4.91 (0.72) |
| Kidney | 9.91 (2.00) | 11.44 (2.76) | 6.00 (2.14) | 3.52 (0.77) |
| Intestine | 3.06 (1.11) | 6.59 (3.43) | 10.34 (4.91) | 14.04 (2.87) |
| Spleen | 2.37 (0.41) | 1.28 (0.19) | 0.86 (0.14) | 0.75 (0.17) |
| Lung | 6.86 (0.65) | 4.44 (0.39) | 2.41 (0.66) | 2.94 (0.62) |
| Stomach*^{b}* | 1.30 (0.43) | 2.53 (0.97) | 2.82 (1.88) | 2.26 (0.88) |
| Pancreas | 4.83 (0.60) | 1.81 (0.15) | 0.83 (0.10) | 0.77 (0.28) |
| Heart | 5.07 (0.47) | 2.17 (0.34) | 0.98 (0.21) | 0.84 (0.37) |
| Brain | 3.17 (0.45) | 1.22 (0.09) | 0.32 (0.02) | 0.24 (0.05) |
| | [¹²⁵I]14 | | | |
| Blood | 3.64 (0.78) | 4.38 (0.52) | 2.87 (0.82) | 2.35 (1.38) |
| Liver | 9.17 (3.45) | 8.48 (2.84) | 7.64 (2.19) | 5.85 (1.09) |
| Kidney | 10.87 (2.95) | 13.12 (4.30) | 12.05 (5.44) | 4.95 (1.82) |
| Intestine | 3.12 (0.85) | 5.06 (1.51) | 15.47 (5.48) | 18.30 (7.21) |
| Spleen | 2.29 (0.36) | 1.64 (0.29) | 0.84 (0.29) | 0.92 (0.07) |
| Lung | 9.06 (0.47) | 7.01 (0.28) | 5.83 (0.55) | 5.23 (1.44) |
| Stomach*^{b}* | 1.59 (0.71) | 2.94 (0.86) | 4.01 (2.29) | 3.49 (2.00) |
| Pancreas | 4.96 (0.44) | 2.01 (0.49) | 1.22 (0.21) | 0.87 (0.30) |
| Heart | 6.77 (0.74) | 2.69 (0.45) | 1.30 (0.41) | 0.98 (0.25) |
| Brain | 4.57 (0.27) | 1.51 (0.17) | 0.49 (0.06) | 0.26 (0.03) |
| | [¹²⁵I]17 | | | |
| Blood | 3.16 (0.82) | 1.51 (0.23) | 0.95 (0.19) | 0.70 (0.60) |
| Liver | 6.87 (2.18) | 5.16 (0.73) | 2.76 (0.40) | 1.86 (0.83) |
| Kidney | 7.26 (2.18) | 7.00 (1.49) | 4.93 (1.52) | 2.43 (1.17) |
| Intestine | 1.59 (0.51) | 4.70 (1.46) | 11.67 (3.56) | 9.02 (3.14) |
| Spleen | 1.45 (0.56) | 0.74 (0.10) | 0.48 (0.10) | 0.43 (0.14) |
| Lung | 6.71 (1.39) | 1.76 (0.29) | 0.97 (0.20) | 0.75 (0.33) |
| Stomach*^{b}* | 0.68 (0.33) | 1.06 (0.66) | 0.48 (0.09) | 0.96 (0.46) |
| Pancreas | 2.83 (0.75) | 0.77 (0.17) | 0.29 (0.14) | 0.16 (0.05) |
| Heart | 3.84 (1.04) | 1.06 (0.10) | 0.37 (0.09) | 0.25 (0.12) |
| Brain | 1.69 (0.43) | 0.54 (0.12) | 0.11 (0.05) | 0.03 (0.02) |
| | [¹²⁵I]20 | | | |
| Blood | 3.14 (0.73) | 2.77 (0.28) | 1.75 (0.38) | 0.87 (0.28) |
| Liver | 6.05 (1.49) | 6.63 (1.08) | 4.23 (0.63) | 4.45 (3.14) |
| Kidney | 11.08 (2.96) | 11.22 (2.26) | 5.82 (1.11) | 2.43 (1.04) |
| Intestine | 2.11 (0.75) | 6.12 (0.83) | 12.67 (2.13) | 14.87 (5.42) |
| Spleen | 2.18 (0.48) | 1.36 (0.15) | 0.69 (0.14) | 0.42 (0.02) |
| Lung | 6.69 (0.86) | 3.14 (0.23) | 1.62 (0.22) | 0.80 (0.19) |
| Stomach*^{b}* | 1.30 (0.43) | 2.53 (0.97) | 2.82 (1.88) | 2.26 (0.88) |
| Pancreas | 5.28 (0.99) | 2.65 (0.64) | 0.92 (0.19) | 0.36 (0.09) |
| Heart | 6.23 (0.63) | 2.57 (0.41) | 0.98 (0.14) | 0.42 (0.09) |
| Brain | 3.07 (0.39) | 1.48 (0.19) | 0.37 (0.07) | 0.14 (0.12) |

| | | | | |
|---|---|---|---|---|
| *^{a}* Expressed as % injected dose per gram. Each value represents the mean for 3-5 animals. Values in parentheses are standard deviations. *^{b}* Expressed as % injected dose per organ. | | | | |

### (4) Autoradiography Using Brain Tissue Sections from Alzheimer's Disease Patients

Fig. 6 shows the results of autoradiography and immunostaining using hippocampal sections from Alzheimer's disease patients. In this Figure, panel A shows the results of autoradiography with aurone derivatives. Panels B, C, D and E show the results of immunostaining with anti-Aβ42 antibody. In the site E where accumulation of radioactivity was not observed, deposition of senile plaques by immunostaining was not recognized. On the other hand, in the sites B, C and D where intensive radioactivity was observed in autoradiography, deposition of senile plaques by immunostaining was recognized. These results demonstrated that aurone derivatives have a selective binding property to senile plaques on brain sections from Alzheimer's disease patients.

### EXAMPLE 2

### Experimental Methods

### (1) Reagents and Instruments

The same reagents and instruments as used in Example 1 were used.

### (2) Synthesis ofAurone Derivatives

### Synthesis of 2-((ethoxycarbonyl)methoxy)-5-iodobenzoic acid methyl ester (Compound 21)

To a solution of 2-hydroxy-5-iodobenzoic acid methyl ester (2.0 g, 7.19 mmol) in acetone (10 mL), K₂CO₃ (2.7 g) was added. Subsequently, ethylbromoacetate (2.0 mL) was added thereto dropwise under stirring. The mixture was heated to reflux for 3 h. After completion of the reaction, the solvent was evaporated. The residue was dissolved in purified water (100 mL) and extracted with ethyl acetate (100 mL). The extract was dried over anhydrous sodium sulfate, and evaporation of the solvent gave Compound 21. Yield: 2.62 g (yield rate: 99 %) ¹H NMR (300 MHz, CDCl₃) · 1.29 (t, J = 7.2 Hz, 3H), 3.90 (s, 3H), 4.25 (q, J = 6.0 Hz, 2H), 4.69 (s, 2H), 6.66 (d, J = 8.7 Hz, 1H), 7.71 (dd, J = 2.4, 2.4 Hz, 1H), 8.12 (d, J = 2.1 Hz, 1H).

### Synthesis of 2-(carboxymethoxy)-5-iodobenzoic acid (Compound 22)

To a solution of Compound 21 (2.62 g, 7.19 mmol) in methanol (2 mL), 10% KOH (3.0 mL) was gradually added thereto. The mixture was reacted for 2h. After completion of the reaction, 1 N HCl was added under cooling. The formed crystals were vacuum filtered to give Compound 22. Yield: 2.02 g (yield rate: 87.2 %).

### Synthesis of 5-iodobenzofuran-3-ylacetate (Compound 23)

Compound 22 (2.02 g, 6.27 mmol) was dissolved in a mixture of acetic anhydride (30 mL) and acetic acid (6 mL). Then, sodium acetate (1.5 g) was added thereto, and the mixture was heated to reflux for 24 h. After completion of the reaction, the reaction mixture was dissolved in 100 ml of purified water and extracted with 200 mL of ethyl acetate, to thereby obtain Compound 23. Yield: 1.45 g (yield rate: 76.6 %) ¹H NMR (300 MHz, CDCl₃)·2.37 (s, 3H), 7.23 (d, J = 8.1 Hz, 1H), 7.59 (dd, J = 1.5, 1.8 Hz, 1H), 7.90 (d, J = 1.5 Hz, 1H), 7.98 (s, 1H).

### Synthesis of 5-iodobenzofuran-3(2H)-one (Compound 24)

To a solution of Compound 23 (1.45 g, 4.80 mmol) in methanol (18 mL), purified water (10 mL) and IN HCl (4 mL) were added. The mixture was heated to reflux for 3 h under stirring. After completion of the reaction, the reaction mixture was cooled and purified water (100 mL) was added thereto. The resultant crystals were vacuum filtered to thereby obtain Compound 24. Yield: 1.17 g (yield rate: 93.8 %) ¹H NMR (300 MHz, CDCl₃)·4.65 (s, 2H), 6.96 (d, J = 9.0 Hz, 1H), 7.85 (dd, J = 1.8, 1.8 Hz, 1H), 7.99 (d, J = 1.8 Hz, 1H).

### Synthesis of (Z)-2-(4-hydroxybenzylidene)-5-iodobenzofuran-3(2H)-one (Compound 25)

To a solution of Compound 24 (50 mg, 0.1922 mmol) and 4-hydroxybenzaldehyde (30 mg, 0.246 mmol) in chloroform, aluminum (1.7 g) was added. Then, the mixture was stirred for 10 h. After completion of the reaction, the reaction mixture was extracted with methanol (100 mL). Evaporation of the solvent gave Compound 25. Yield: 78 mg (yield rate: 87.0 %) ¹H NMR (300 MHz, CDCl₃)·6.91 (s, 1H), 6.99 (d, J = 6.9 Hz, 2H), 7.14 (d, J = 8.4 Hz, 1H), 7.85-7.90 (m, 3H), 8.11 (d, J =2.1 Hz, 1H).

### Synthesis of (Z)-2-(4-(2-hydroxyethoxy)benzylidene)-5-iodobenzofuran-3(2H)-one (Compound 26)

To a solution of Compound 25 (194 mg, 0.533 mmol) and ethylene chlorohydrin (0.1 mL, 1.43 mmol) in dimethylsulfoxide (7 mL), potassium carbonate (3.7 g) was added. The mixture was heated and stirred for 15 h. After completion of the reaction, the reaction mixture was extracted with ethyl acetate (100 mL). After the solvent was evaporated, the residue was subjected to silica gel column chromatography using ethyl acetate as an elution solvent, to thereby obtain Compound 26. Yield: 123 mg (yield rate: 56.5 %) ¹H NMR (300 MHz, CDCl₃)·2.01 (s, 1H), 4.02 (s, 2H), 4.18 (t, J = 3.9 Hz, 2H), 6.89 (s, 1H), 7.00 (d, J = 7.5 Hz, 2H), 7.14 (d, J =8.4 Hz, 1H), 7.80-7.92 (m, 3H), 8.12 (d, J =1.8 Hz, 1H).

### Synthesis of (Z)-2-(4-(2-(2-hydroxyethoxy)ethoxy)benzylidene)-5-iodobenzofuran-3(2H)-one (Compound 27)

To a solution of Compound 25 (150 mg, 0.412 mmol) and ethylene glycol mono-2-chloroethyl ether (0.15 mL, 1.42 mmol) in dimethylsulfoxide (7 mL), potassium carbonate (3.7 g) was added. The mixture was heated and stirred for 5 h. After completion of the reaction, the reaction mixture was extracted with ethyl acetate (200 mL). After the solvent was evaporated, the residue was subjected to silica gel column chromatography using ethyl acetate as an elution solvent, to thereby obtain Compound 27. Yield: 56 mg (yield rate: 30.2 %) ¹H NMR (300 MHz, CDCl₃)·2.17 (s, 1H), 3.70 (d, J = 4.8 Hz, 2H), 3.78 (d, J = 4.8 Hz, 2H), 3.92 (t, J = 4.8 Hz, 2H), 4.21 (t, J =4.5Hz, 2H), 6.89 (s, 1H), 7.00 (d, J = 11.4 Hz, 2H), 7.13 (d, J =8.7 Hz, 1H), 7.84-7.91 (m, 3H), 8.11 (d, J =2.1 Hz, 1H).

### Synthesis of (Z)-2-(4-(2-(2-(2-hydroxyethoxy)ethoxy)ethoxy)benzylidene)-5-iodobenzofuran-3(2H)-one (Compound 28)

To a solution of Compound 25 (150 mg, 0.412 mmol) and 2-[2-(2-cloroethoxy)ethoxy]ethanol (0.1 mL, 0.69 mmol) in dimethylsulfoxide (7 mL), potassium carbonate (3.7 g) was added. The mixture was heated and stirred for 6 h. After completion of the reaction, the reaction mixture was extracted with ethyl acetate (200 mL). The solvent was evaporated and the residue was subjected to silica gel column chromatography using ethyl acetate as an elution solvent, to thereby obtain Compound 28. Yield: 140 mg (yield rate: 67.9 %).

### Synthesis of (Z)-2-(4-(2-hydroxyethoxy)benzylidene)-5-(tributyltin)benzofuran-3(2H)-one (Compound 29)

To a solution of Compound 26 (20 mg, 0.048 mmol) in dioxane (3 mL), bis(tributyltin) (0.4 mL), tetra-triphenylphosphinepalladium (50 mg, 0.043 mmol) and triethylamine (3 mL) were added. The mixture was heated at 90°C for 5 h. After the solvent was evaporated, the residue was subjected to silica gel column chromatography with an elution solvent of ethyl acetate/hexane (1/1) to thereby obtain Compound 29. Yield: 7.0 mg (yield rate: 25.0 %) ¹H NMR (300 MHz, CDCl₃)·0.86-1.61 (m, 27H), 2.06 (s, 1H), 4.02 (s, 2H), 4.16 (t, J = 3.6 Hz, 2H), 6.88 (s, 1H), 6.99 (d, J = 9.0 Hz, 2H), 7.30 (d, J =12.3 Hz, 1H), 7.73 (d, J =9.0 Hz, 1H), 7.90-7.92 (m, 3H).

### Synthesis of (Z)-2-(4-(2-(2-hydroxyethoxy)ethoxy)benzylidene)-5-(tributyltin)benzofuran-3(2H)-one (Compound 30)

To a solution of Compound 27 (40 mg, 0.089 mmol) in dioxane (7 mL), bis(tributyltin) (0.4 mL), tetra-triphenylphosphinepalladium (50 mg, 0.043 mmol) and triethylamine (4 mL) were added. The mixture was heated at 90°C for 7 h. After the solvent was evaporated, the residue was subjected to silica gel column chromatography using ethyl acetate as an elution solvent to thereby obtain Compound 30. Yield: 17 mg (yield rate: 31.4 %) ¹H NMR (300 MHz, CDCl₃₎ ·0.86-1.32 (m, 27H), 2.11 (s, 1H), 3.70 (t, J = 4.8 Hz, 2H), 3.78 (t, J = 4.8 Hz, 2H), 3.91 (t, J = 4.5 Hz, 2H), 4.22 (t, J =5.1 Hz, 2H), 6.89 (s, 1H), 7.00 (d, J = 7.2 Hz, 2H), 7.31 (d, J =8.1 Hz, 1H), 7.71 (d, J =9.0 Hz, 1H), 7.88-7.91 (m, 3H).

### Synthesis of (Z)-2-(4-(2-(2-(2-hydroxyethoxy)ethoxy)ethoxy)benzylidene)-5-(tributyltin)benzofuran-3(2H)-one (Compound 31)

To a solution of Compound 28 (10 mg, 0.02 mmol) in dioxane (5 mL), bis(tributyltin) (0.3 mL), tetra-triphenylphosphinepalladium (40 mg, 0.034 mmol) and triethylamine (4 mL) were added. The mixture was heated at 90°C for 10 h. After the solvent was evaporated, the residue was subjected to silica gel column chromatography using ethyl acetate as an elution solvent, to thereby obtain Compound 31. Yield: 4 mg (yield rate: 30.3 %)¹H NMR (300 MHz, CDCl₃)·0.86-1.59 (m, 27H), 2.11 (s, 1H), 3.63 (t, J = 3.3 Hz, 2H), 3.73-3.75 (m, 4H), 3.90 (t, J = 4.2 Hz, 2H), 4.21 (t, J =4.2 Hz, 2H), 6.88 (s, 1H), 7.10 (d, J = 8.7 Hz, 2H), 7.31 (d, J =8.1 Hz, 1H), 7.64-7.70 (m, 3H), 7.90 (d, J =4.8 Hz, 1H).

### (3) Labeling of Aurone Derivatives with ¹²⁵I

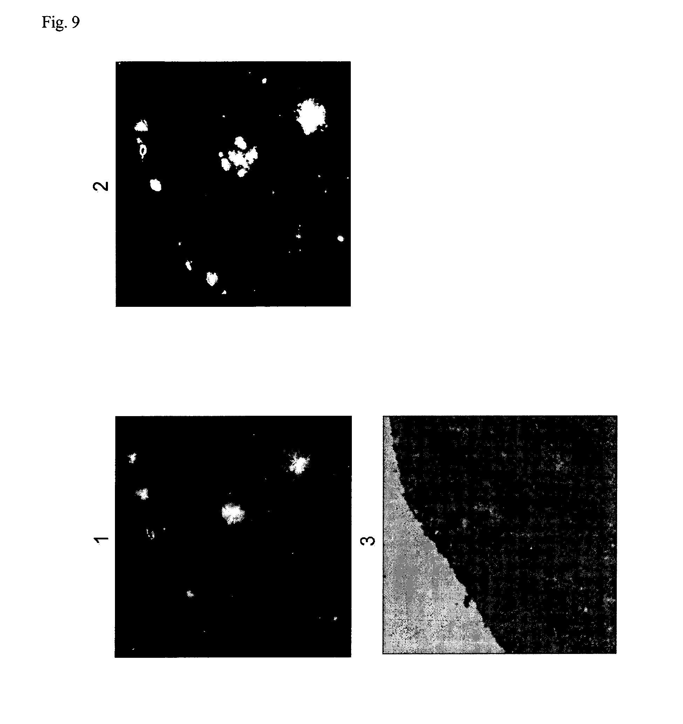

[¹²⁵I]NaI (1 to 5 mCi) and 1 N hydrochloric acid (50 mL) were added to solutions of various tributyltin compounds (Compounds 29, 30 and 31) (1 mg/mL) in ethanol (50 mL), and 3% (w/v) aqueous hydrogen peroxide (50 mL) was added finally (Figure 9). The mixture was allowed to stand for 1 min at room temperature, then saturated aqueous sodium hydrogen sulfite (100 mL) was added to terminate the reaction, and saturated aqueous sodium hydrogen carbonate (100 mL) was added to neutralize the reaction solution. The mixture was extracted with ethyl acetate, dehydrated by passing through a Pasteur pipette containing sodium sulfate, and then purified by reversed-phase HPLC (water:acetonitrile = 3:7). Nonradioactive compounds were analyzed for absorbance at 254 nm by HPLC as standards. Then, labeled compounds having matching absorbances were separated and extracted with ethyl acetate, followed by evaporation of ethyl acetate under nitrogen gas flow.

### (4) Preparation of Ab(1-42) aggregates

Ab(1-42) was dissolved in 10 mM phosphate buffer containing 1 mM EDTA (pH 7.4) to give a concentration of 0.25 mg/mL, and the mixture was incubated at 37°C for 42 h to thereby prepare Ab(1-42) aggregates.

### (5) Calculation of Inhibition Constant (Kᵢ values) by Binding Inhibition Experiment Using Ab(1-42) Aggregates

850 mL of 10% EtOH solution, 50 mL of a solution of [¹²⁵I](Z)-2-(4-aminobenzylidene)-5-iodobenzofuran-3(2H)-one in 10% ethanol, and 50 mL of a sample solution at various concentrations (0 to 20 mM) were mixed. Finally, 50 mL of Ab(1-42) aggregate solution was added thereto, and the mixture was allowed to stand at room temperature for 3 h. Ab(1-42) aggregate-bound compounds and nonbound compounds were separated with a Brandel M-24R cell harvester using a Whatman GF/B filter. Radioactivities remaining on the filter were measured with a γ-counter, and inhibition curves were created using Graph Pad Prism 4.0 to calculate inhibition constants (Kᵢ values).

### (6) In vivo Radioactivity Distribution Experiment in Normal Mice

¹²⁵I-labeled compounds (Compounds 26, 27 and 28) were diluted with physiological saline containing 10% ethanol. To each of 5-week-old male ddY mice (5 animals/group), 100 µL of the labeled compound (0.3 to 0.5 µCi) was injected intravenously. Animals were decapitated at 2, 10, 30 or 60 min after the injection, and the blood was collected. Then, major organs were removed, and weights and radioactivities thereof were measured.

### (7) Fluorescence Staining and Immunostaining on Brain Sections from Transgenic Mice

As Alzheimer's disease model mouse, Tg2576 mice (20-month-old) were used. Brain tissues were removed and frozen in carboxymethyl cellulose (4%). Then, a series of sections 10 µm thick were prepared therefrom. The thus prepared sections were reacted with a ligand (a solution of Compound 26 and thioflavin in 50% EtOH) for 3 min. Then, the sections were washed with 50% EtOH (1 min x twice) and observed under a fluorescence microscope. Further, using adjacent sections, immunostaining was performed by conventional methods with anti-amyloid β(1-42) antibody.

### (8) In vitro Autoradiography and Immunostaining on Brain Sections from Alzheimer's Disease Patients

Hippocampal sections (5 µm thick) from Altzheimer's disease patients were dewaxed by washing with xylene ((5 min x 2) and with 100% EtOH, 100% EtOH, 95% EtOH, 85% EtOH and 70% EtOH (1 min x 1 for each). After air-drying, the sections were reacted with a ligand (40% EtOH solution) for 1 h. Then, the sections were washed with saturated aqueous lithium carbonate solution in 40% EtOH (2 min x 2), with 40% EtOH (2 min x 2) and with water (30 sec x 1), and immobilized on imaging plates for 24 h. Subsequently, the sections were analyzed with BAS2500. Further, using adjacent sections, immunostaining was performed by conventional methods with anti-amyloid β(1-42) antibody.

### [Experimental Results]

### (1) Synthesis of Aurone Derivatives

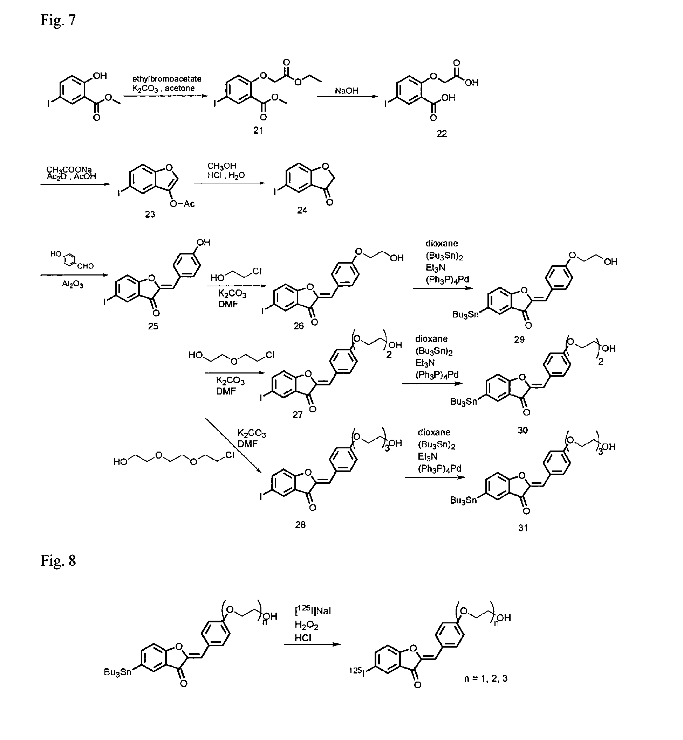

Fig. 7 shows synthetic pathways for aurone derivatives. The aurone skeleton was formed by an aldol condensation reaction. Individual iodinated compounds were converted to tributyltin forms through a reaction with bis(tributyltin) using palladium as a catalyst. These tributyltin compounds were used as labeling precursor compounds in the labeling with radioactive iodine.

### (2) ¹²⁵I Labeling Experiment of Aurone Derivatives

Fig. 8 shows a ¹²⁵I labeling pathway for aurone derivatives. ¹²⁵I labeling was performed with hydrogen peroxide as an oxidizing agent and through tin-iodine exchange reaction, to thereby obtain ¹²⁵I-labeled compounds of interest. Absorbances at 254 nm of non-radioactive compounds were analyzed by reversed-phase HPLC in advance. By isolating and purifying those labeled compounds with matching retention time, ¹²⁵I-labeled compounds of interest were obtained with a radiochemical purity of 95% or more.

### (3) Examination concerning the Binding Affinity of Aurone Derivatives to Ab(1-42) Aggregates

In order to calculate inhibition constants (Kᵢ values) for various aurone derivatives synthesized, binding inhibition experiments were performed using [¹²⁵I](Z)-2-(4-aminobenzylidene)-5-iodobenzofuran-3(2H)-one as a radioactive ligand. The Kᵢ values obtained by the experiments are shown in Table 14. The binding affinity of aurone derivatives to Ab(1-42) aggregates is within the range from 1.05 to 3.36 nM in Kᵢ values. Thus, aurone derivatives have shown a high binding property to amyloid aggregates. No difference in binding property resulting from the length of introduced ethyleneoxy chains was observed.

**Table 14**

| *In vitro* Binding Inhibition Experiment Using Aβ 42 Aggregates | |
|---|---|
| Compound | Ki (nM) |
| 26 | 1.05 ± 0.06 |
| 27 | 3.36 ± 0.29 |
| 28 | 2.56 ± 0.31 |

### (4) In vivo Radioactivity Distribution Experiment of ¹²⁵I-Labeled Aurone Derivatives in Normal Mice

*In vivo* radioactivity distribution experiments of ¹²⁵I-labeled aurone derivatives were performed using 5-week-old normal mice. The results of biodistribution of radioactivity after injection of [¹²⁵I] Compound 26, [¹²⁵I] Compound 27 and [¹²⁵I] Compound 28 into mice from the tail vein are shown in Table 15. All of [¹²⁵I] Compound 26, [¹²⁵I] Compound 27 and [¹²⁵I] Compound 28 have shown high migration to the brain at 2 min after the injection (2.4 to 4.5% ID/g). Further, the radioactivity remaining in the brain at 30 min after injection was as low as 0.08 to 0.16% ID/g. From these results, it has become clear that these three types of aurone derivatives show rapid clearance of radioactivity from normal brain. Besides, no remarkable radioactivity stasis in the blood was observed.

**Table 15**

| Radioactivity Distribution of Aurone Derivatives in the Brain and Blood of Normal Mice | | | | |
|---|---|---|---|---|
| [125I]26 | Time (min) after Injection | | | |
| | 2 | 10 | 30 | 60 |
| Brain | | | | |
| Mean | 4.51 | 1.49 | 0.24 | 0.09 |
| Standard Deviation | 0.25 | 0.28 | 0.03 | 0.04 |
| Blood | | | | |
| Mean | 4.97 | 3.88 | 2.38 | 1.24 |
| Standard Deviation | 0.96 | 1.09 | 0.85 | 0.35 |
| | | | | |

| [125I]27 | Time (min) after Injection | | | |
|---|---|---|---|---|
| | 2 | 10 | 30 | 60 |
| Brain | | | | |
| Mean | 3.69 | 1.53 | 0.38 | 0.16 |
| Standard Deviation | 0.22 | 0.31 | 0.05 | 0.03 |
| Blood | | | | |
| Mean | 3.61 | 5.18 | 1.11 | 0.68 |
| Standard Deviation | 0.74 | 2.54 | 0.73 | 0.45 |
| | | | | |

| [125I]28 | Time (min) after Injection | | | |
|---|---|---|---|---|
| | 2 | 10 | 30 | 60 |
| Brain | | | | |
| Mean | 2.81 | 0.84 | 0.18 | 0.08 |
| Standard Deviation | 0.36 | 0.12 | 0.02 | 0.02 |
| Blood | | | | |
| Mean | 4.75 | 2.70 | 1.13 | 0.55 |
| Standard Deviation | 0.79 | 0.23 | 0.29 | 0.05 |

### (5) Fluorescence Staining and Immunostaining on Brain Sections from Transgenic Mice

Fluorescence staining was performed on brain sections from Alzheimer's disease model mouse (Tg2576) utilizing the fluorescence property of Compound 26 (Fig. 9). As a result, the fluorescence image of Compound 26 was consistent with the fluorescence image of thioflavin S on adjacent section and the immunostaining image of anti-amyloid antibody on adjacent section. From these results, it was demonstrated that Compound 26 possesses a binding property not only to synthetic amyloid β42 aggregates (*in vitro* binding experiment) but also to senile plaques in mice.

### (6) In vitro Autoradiography and Immunostaining on Brain Sections from Alzheimer's Disease Patients

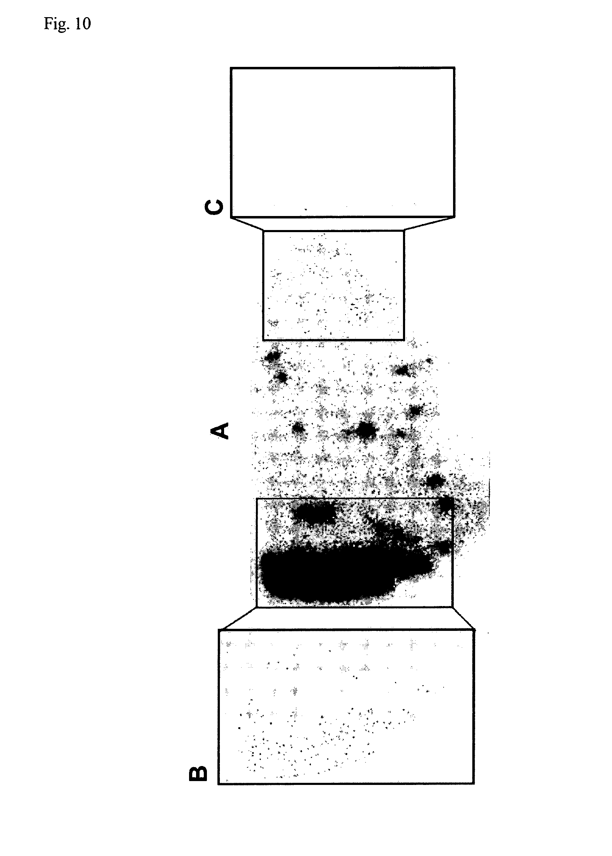

Autoradiography was performed on brain sections from Altzheimer's disease patients with [¹²⁵I]26 (Fig. 10). While the radioactivity of [¹²⁵I]26 was observed at the positive site of immunostaining with anti-amyloid antibody, no accumulation of radioactivity was observed in sites without senile plaques. From these results, it was suggested that [¹²⁵I]26 has a binding property to the senile plaque amyloid accumulating in Altzheimer's disease patients.

The present specification encompasses the contents disclosed in the specifications and/or the drawings of Japanese Patent Applications (No. 2006-328131 and No. 2007-081602) based on which the present patent application claims priority. All publications, patents and patent applications cited herein are incorporated herein by reference in their entirety.

## Claims

1. A composition for diagnosing amyloid-related diseases, comprising a compound represented by general formula (I): 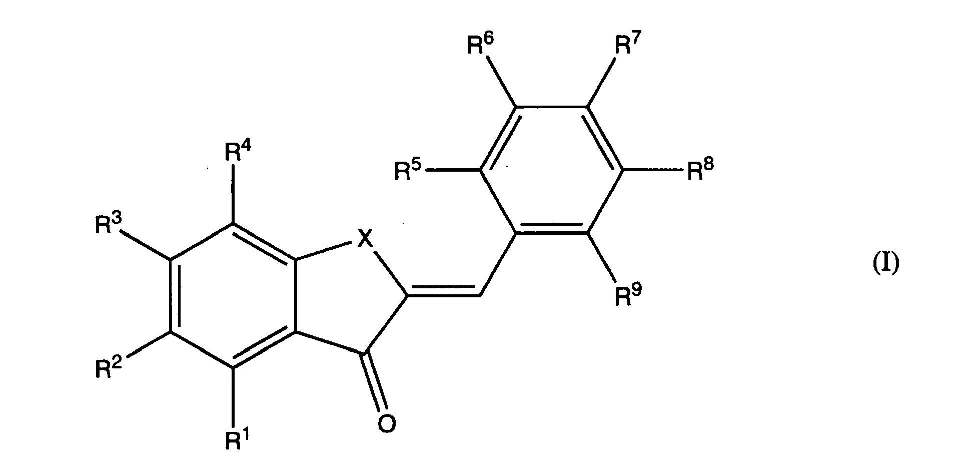 wherein X represents O or NH; R¹, R², R³ and R⁴ are the same or different and each represent a hydrogen atom, a halogen atom, a group represented by a formula -(CH₂CH₂O)ₙ-F where n is an integer from 1 to 10 or a group represented by a formula -(CH₂CH₂O)ₙ-OH where n is an integer from 1 to 10; and R⁵, R⁶, R⁷, R⁸ and R⁹ are the same or different and each represent a hydrogen atom, a halogen atom, a dimethylamino group, a methylamino group, an amino group, a methoxy group, a hydroxyl group, a group represented by a formula -(CH₂CH₂O)ₙ-F where n is an integer from 1 to 10 or a group represented by a formula -(CH₂CH₂O)ₙ-OH where n is an integer from 1 to 10;
or said compound labeled with a radionuclide, or a pharmaceutically acceptable salt of the compound or the labeled compound.

2. The composition according to claim 1, wherein X in general formula (I) is O.

3. The composition according to claim 1 or 2, wherein R² in general formula (I) is a fluorine atom, an iodine atom or a group represented by a formula -(CH₂CH₂O)ₙ-F where n is an integer from 1 to 10.

4. The composition according to claim 1 or 2, wherein R² in general formula (I) is a fluorine atom, an iodine atom or a group represented by a formula -(CH₂CH₂O)ₙ-F where n is an integer from 1 to 10; and R¹, R³ and R⁴ each represent a hydrogen atom.

5. The composition according to any one of claims 1 to 4, wherein R⁷ in general formula (I) is a fluorine atom, an iodine atom, a dimethylamino group, a methylamino group, an amino group, a methoxy group, a hydroxyl group, a group represented by a formula -(CH₂CH₂O)ₙ-F where n is an integer from 1 to 10 or a group represented by a formula -(CH₂CH₂O)ₙ-OH where n is an integer from 1 to 10.

6. The composition according to any one of claims 1 to 4, wherein R⁷ in general formula (I) is a fluorine atom, an iodine atom, a dimethylamino group, a methylamino group, an amino group, a methoxy group, a hydroxyl group, a group represented by a formula -(CH₂CH₂O)ₙ-F where n is an integer from 1 to 10 or a group represented by a formula -(CH₂CH₂O)ₙ-OH where n is an integer from 1 to 10; and R⁵, R⁶, R⁸ and R⁹ each represent a hydrogen atom.

7. The composition according to any one of claims 1 to 6, wherein the radionuclide is a positron-emitting radionuclide.

8. The composition according to any one of claims 1 to 6, wherein the radionuclide is a γ-ray-emitting radionuclide.

9. The composition according to any one of claims 1 to 8, wherein the amyloid-related disease is Alzheimer's disease.

10. A method of screening for therapeutic or prophylactic agents for amyloid-related diseases, comprising a step of administering a test substance to an amyloid-related disease model animal, a step of administering the composition according to any one of claims 1 to 9 to said model animal, and a step of examining the distribution or quantity of the compound represented by general formula (I) contained in the brain of said model animal.

11. A method of evaluating therapeutic or prophylactic agents for amyloid-related diseases, comprising a step of administering a therapeutic or prophylactic agent for amyloid-related diseases to an amyloid-related disease model animal, a step of administering the composition according to any one of claims 1 to 9 to said model animal, and a step of examining the distribution or quantity of the compound represented by general formula (I) contained in the brain of said model animal.
